# EUROPEAN PATENT APPLICATION

(11) **EP 2 554 180 A1**
(43) Date of publication of application: **06.02.2013**
(21) Application number: 11758862.4
(22) Date of filing: 09.03.2011
(51) Int. Cl.: A61K 38/08, C07K 7/06, A61P 25/14, A61P 21/00

(54) **COMPOUNDS FOR USE IN THE TREATMENT OF DISEASES BASED ON THE EXPRESSION OF TOXIC TRANSCRIPTS**

(30) Priority: 26.03.2010 ES 201030462
(71) Applicant: Universitat de Valéncia, 46010 Valencia (ES); Fundación De La Comunidad Valenciana Centro De Investigacion Principe Felipe, 46012 Valencia (ES); Consejo Superior De Investigaciones Científicas, 28006 Madrid (ES)
(72) Inventor: ARTERO ALLEPUZ, Ruben Darío, E-46100 Valencia (ES); GARCÍA LÓPEZ, Amparo, E-46100 Valencia (ES); ORZÁEZ CALATAYUD, María del Mar, E-46012 Valencia (ES); LLAMUSÍ TROISI, María Beatriz, E-46100 Valencia (ES); PÉREZ PAYÁ, Enrique, E-46012 Valencia (ES); PÉREZ ALONSO, Manuel, E-46100 Valencia (ES)
(74) Representative: Illescas, Manuel
(86) International application number: PCT/ES2011/070159
(87) International publication number: WO 2011/117448

(57) **Abstract**

Compounds to be used in the treatment of diseases based on the expression of toxic transcripts. The present invention relates to peptide molecules, specifically hexapeptides, designed for the prevention and/or treatment of diseases the etiology whereof is based on the presence of toxic transcripts that comprise CUG, CCUG, CGG, CAG and AAG repeats, preferably: DM1 SCA8, DM2, FXTAS, HD and FA.

## Description

### FIELD OF THE INVENTION

The present invention relates to compounds that comprise hexapeptides, to be used in the prevention and/or treatment of diseases the etiology whereof is based on the expression of toxic transcripts which comprise CUG, CCUG, CGG, CAG and AAG repeats, such as, for example, Myotonic Dystrophy type 1 (DM1), Spinocerebellar ataxia type 8 (SCA8), Myotonic Dystrophy type 2 (DM2), Fragile X-associated tremor/ataxia syndrome (FXTAS), Huntington's disease (HD) and Friedreich's Ataxia (FA), respectively.

Therefore, the present invention may be included in the field of medicine in general and, more specifically, in the field related to the prevention and/or treatment of hereditary genetic diseases.

### STATE OF THE ART

DM1, or Steinert's disease (DM1, OMIM #160900) is the most frequent type of muscular dystrophy in the adult population, with a worldwide prevalence of approximately 1 patient per 8,000 people, and is classified as a rare disease. It is a neuromuscular disease, with defining symptoms that primarily affect the muscles, such as myotonia and muscle weakness, although it is characteristically multi-systemic, affecting, amongst other organs and systems, the cardiac system (cardiac arrythmias), the ocular system (cataracts), the endocrine system (hyperinsulinemia) and the reproductive system (hypogonadism).

At the genetic level, DM1 presents an autosomal dominant inheritance pattern, high penetrance and variable expressivity. The origin of the disease lies in a dynamic mutation in the untranslated 3' region (3'UTR) of the dystrophia myotonica protein kinase gene (DMPK, Entrez #1760), located in the 19q13.2-q13.3 chromosomal region. Said mutation consists of an abnormal expansion of repeats of the CTG trinucleotide in exon 15 of said DMPK gene, which in the healthy population appears in a variable number of between 5-35 copies, whereas in patients the number is greater than 50. The number of CTG triplets is correlated with the severity of the symptoms and the age whereat these appear. Thus, in those cases wherein the number of repeats ranges between 50 and a few hundreds, it is referred to as adult-onset DM1 and the first clinical signs usually appear during the second decade of life. In cases wherein the number of repeats is greater, even reaching thousands of copies, the pathology appears from birth in the form of congenital DM (CDM or Thomsen's disease), which is the most severe variant of the disease, with symptoms such as mental retardation, respiratory disorders and muscle differentiation problems, amongst others.

In recent years, a number of hypotheses have been proposed in relation to the molecular mechanisms associated with the pathogenesis of DM1, and RNA toxic gain-of-function is amongst those with the greatest consensus. According to this mechanism, RNAs carrying CUG expansions form hairpin loops that are toxic for those cells that express them, causing, amongst other molecular alterations, changes in the alternative splicing of defined transcripts, by sequestering the regulatory factors thereof. The work of Mankodi *et al.* (2000) on transgenic mice provides decisive support for this hypothesis. Said mice express approximately 250 CUG trinucleotide repeats in a heterologous mRNA, and develop myotonia and muscular defects typical of DM1, which demonstrates that CUG expansions exert a toxic effect by themselves, regardless of their context (Mankodi *et al.,* 2000). Subsequently, other models generated in *Drosophila* confirmed the toxicity of the CUG repeats independently from *DMPK* (Garcia-Lopez *et al.,* 2008).

Since the discovery that RNAs with CUG expansions may be toxic for cells, a whole series of scientific evidence supports the hypothesis that these expansions, or similar expansions, in other RNAs, may cause hereditary genetic pathologies similar to DM1. For example, in DM2, expansions of the CCUG tetranucleotide in the first intron of the RNAs transcribed from the ZNF9 (zinc-finger protein 9, Entrez #7555) gene trigger a pathology that is very similar to DM1 (OMIM #602668). In fact, in the state of the art genes related to the nervous function are known to exist which present altered levels in both DM1 and DM2 patients, which supports the idea of a pathogenesis mechanism common to both diseases.

On the other hand, spinocerebellar ataxia type 8 (SCA8, OMIM #603680) is a neurodegenerative disease caused by the expansion of CTG triplets in a non-coding transcript of the SCA8 gene. It has been observed that the bidirectional transcription of this gene leads to both the expression of RNAs with CUG expansions (untranslated), which trigger molecular alterations related to those described for DM1, and transcripts with CAG expansions that are translated into proteins with polyglutamines.

In the nucleus, RNAs carrying CUG expansions fold to form a hairpin-loop structure capable of binding and sequestering RNA-binding factors, forming large ribonucleoprotein inclusions. Thus far, it has been disclosed that CUG repeats interfere with the activity of an increasing number of nuclear proteins, which include transcription factors and alternative splicing factors. The latter include the hnRNP F and hnRNP H heterogeneous nuclear ribonucleoproteins, as well as proteins belonging to the MBNL1-3 (Muscleblind-like proteins) family. Due to this sequestration, patients present alterations in the alternative processing of hundreds of specific transcripts, which led to the coining of the term spliceopathy, whereof DM1 is the first example described. It has also been proposed that the activity of different microRNAs could be altered.

The sequestration of nuclear proteins prevents them from performing their normal functions in the cell. Several proteins have been isolated and characterised which are capable of binding to double-stranded CUG hairpin loops both *in vitro* and *in vivo.* These include transcription factors such as specificity protein 1 (Sp1), retinoic acid receptor gamma (RARγ) and the members of the family of signal transducers and activators of transcription STAT1 and STAT3. Some of these factors may undergo a sequestration of up to 90% in DM1 cells under culture, which prevents them from activating the transcription of their target genes, thus reducing the expression thereof.

Other transcription factors which are altered in DM1 are NKX2-5 and MyoD. These proteins are related to cardiac development and conduction, and with the differentiation of myoblasts, respectively. The levels of NKX2-5 are increased in patients, whereas MyoD is reduced. The reason why the levels of these transcription factors are de-regulated, as well as their relation to the formation of CUG hairpin loops, is unknown.

Thus far, several works have demonstrated that CUG repeats affect the expression of a large number of genes. Using mouse microarrays, at least 175 muscle transcripts have been detected which are altered by the expression of expanded CUG transcripts. Moreover, at least 128 transcripts are also de-regulated in MBNL1 knockout mice, which suggest that the sequestration and subsequent loss of function of MBNL1 plays a crucial role in the disease.

MBNL1 knockout mice present iridescent-type cataracts, suffer from myotonia and histological defects at the muscular level. MBNL2 knockout mice also develop myotonia (due to an incorrect processing of the Clcn-1 transcripts) and other muscular alterations typical of DM1. Moreover, the overexpression of MBNL1 in model mice that express 250 CTG repeats reverts the defects in the processing of at least 4 transcripts (Serca1, Clcn1, Tnnt3 and ZASP), as well as myotonia. In Drosophila, *mbl* mutant embryos present a lack of organisation of the Z bands of sarcomeres, hypercontraction of the abdomen and alterations in the alternative processing of transcripts such as ZASP, troponin T (tnT) and α-actinin. Likewise, the overexpression of MBNL1 in model flies that express 480 CTG repeats suppresses phenotypes caused by the repeats. All these results entailed a significant change in the study of DM1; thereafter, Muscleblind (Mbl) proteins have been considered to be a decisive element in the development of the disease.

Although DM was first described in 1909, there is still not an effective therapy available. All the treatments applied are palliative and contribute to curb the development of symptoms, but in no case prevent the onset thereof or treat the disease in a definitive manner. Currently, there is no compound capable of reverting lack of chloride conductance so as to reduce myotonia. Some compounds, such as mexityl, quinidine, phenytoin, procainamide or carbamazepine, which inhibit the sodium entry necessary for the initiation and propagation of impulses, are administered to patients in order to treat this symptom. Occasionally, said molecules are used, in turn, as a treatment for cardiac arrhythmias; consequently, given the risk that they entail due to their effect on the cardiac function, it is preferable to avoid using them as anti-myotonic agents. Moreover, many of these treatments reduce muscular strength. Another sodium channel blocker, dehydroepiandrosterone sulfate (DHEAS), has been tested in patients, and it seems to successfully reduce myotonia and cardiac problems, without enhancing muscle weakness. DHEAS is a steroid hormone that is abundantly present in serum and the levels whereof decrease with age. In patients with DM1, this hormone is reduced by up to 60%. Moreover, the effective dose of DHEAS is very high; for this reason, it must be administered by intravenous route, which makes it disadvantageous to use as a chronic treatment. In some cases, creatinine is administered jointly with DHEAS in order to increase muscular strength. However, although the first clinical trials with creatinine in patients showed promising results, subsequent studies are not as positive. Other drugs tested for the treatment of myotonia include tricyclic antidepressants, benzodiazepines, calcium ion antagonists, taurine or prednisone, with contradictory results.

On the basis of the mechanism of action of the disease described above, which proposes the sequestration and subsequent loss of function of Muscleblind proteins as the main trigger of the disease, strategies have been developed to find molecules that inhibit the interaction between MBNL1 and hairpin loops with CUG repeats. Thus, compounds have been identified which are capable of inhibiting said interaction *in vitro.* Molecules with the sequence ((Quin/Pip)-(Asn/Pro)-Cys-Lys) were capable of shifting MBNL1 in the binding to repeats. Moreover, antibiotics pentamidine and neomycin B, as well as ethidium bromide and thiazole orange, inhibited the binding of MBNL1 to the CUG repeats. Furthermore, pentamidine reverted the splicing defects in the IR and TNNT2 transcripts in HeLA cells under culture, and reduced the formation of nuclear inclusions containing MBNL1 by 21 %. In DM1 model mice, pentamidine also improved the splicing of Clcn-1 and Serca1, albeit discreetly and without a clear dose response. Thus, pentamidine is the first example of a molecule identified *in vitro* that has a potential therapeutic effect on CUG repeats *in vivo.* However, this molecule could affect the processing of other target transcripts of MBNL1 in the absence of repeats, which could counteract its long-term therapeutic value. A molecule has also been developed in the state of the art which is based on the three-dimensional structure of CTG and/or CUG repeats, the target whereof were T-T or U-U unpairings. Athough small molecules capable of binding to G-G, C-C or A-A were already known, thus far no compound had been found which bound to T-T or U-U in a selective manner. Thus, it was shown that the ligand formed by triaminotriazine (which interacts with T-T and U-U) plus acridine (intercalating agent) bound to CTG and CUG repeats in a specific manner and with a high affinity as compared to other sequences.

Moreover, the state of the art discloses the design of a pentamer of the compound Hoechst 33258 which is capable of binding to CUG and CAG repeats, and inhibiting the formation of RNA-MBNL1 complexes in both cases. This molecule was permeable and non-toxic at least in mouse myoblasts. Parallel to this, a ligand was also developed with a high affinity for RNA molecules with two pyrimidine-rich internal unpairings, such as those formed by CCUG repeats in DM2. This compound consisted of three modules of 6'-N-5-hexynoate kanamycin A bound to a peptoid skeleton and separated by four spacer monomers. Reducing the number of spacers from four to two caused this molecule to become a ligand with a higher affinity for CUG repeats than CCUG.

These strategies or compounds present in the state of the art make it possible for MBNL1 to be released, thereby reverting the splicing defects. Moreover, when nuclear inclusions are dissipated, there would be more free DMPK transcripts in the cytoplasm to be translated. However, the redistribution of the mutant RNAs could have a new toxic effect. Although the formation of aggregates of CUG and MBNL1 in the cytoplasm of cardiomyocytes does not cause defects in mice, other proteins could be affected in the short or long term. Moreover, those molecules that interfere with the binding between MBNL1 and CUG could also inhibit the binding between MBNL1 and other target transcripts in the nucleus, as has been found for pentamidine, or interfere with other proteins which have an RNA-binding mechanism similar to that of MBNL1. Finally, any therapeutic approach based on MBNL1 has the limitation that not all the toxicity of CUG repeats is due to the sequestration of MBNL1.

The origin of most neuromuscular diseases lies in mutations in a single gene and, therefore, they are good candidates for the development of gene therapies. However, in the case of DM1, the tissues involved are primarily post-mitotic, which entails a disadvantage with respect to most viral vectors. In this regard, it has been proposed that using molecules such as anti-sense oligonucleotides (ASOs) could be advantageous. Anti-sense oligonucleotides do not supply a copy of the gene, but, instead, modulate the products of an existing gene. Currently, several molecules based on this strategy are already in the clinical phase. One example is the case of Duchenne's Muscular Dystrophy (DMD, OMIM #300377), for which the company AVI Biopharma has two molecules in the pre-clinical phase in the United States, one of which is already in the 1b/2 clinical phase in the United Kingdom (www.avibio.com). The use of ASOs in model mice that express 250 CTG repeats, as well as in MBNL1 knockout mice, is known in the state of the art, and the effect thereof on the splicing of exon 7a of Clcn-1 transcripts has been studied. In both models, a single injection in the anterior tibial muscle recovered the normal splicing pattern of the Clcn-1 transcripts for at least three weeks, reverting the myotonia. However, due to the large number of messengers the processing whereof is altered in patients, several ASOs should be combined with different targets in order to effectively treat the different symptoms.

One alternative to the combined use of ASOs is using anti-sense oligonucleotides that act at the level of DMPK transcripts in order to eliminate the source of toxicity. In the state of the art, assays have already been performed which are aimed at the inhibition of the expression of DMPK in patient cells under culture using specific ASOs. However, this approach did not discriminate between mutant transcripts and wild transcripts. Reducing the total expression of DMPK could be equally pathological, given the important role of DMPK in the cardiac function and the metabolism of insulin. Several works in the state of the art have used ASOs made up of CAG repeats (CAG7 or CAG25) in order to direct their effect preferably towards transcripts with long CUG repeats. In both myoblasts under culture and DM1 model mice, these oligonucleotides reverted the splicing defects of Clcn-1, in addition to specifically reducing the levels of the mutant transcript by up to 50%, probably because the binding between short CAG repeat sequences and toxic RNAs promotes the formation of heteroduplexes without unpairings; this prevents the sequestration of proteins such as MBNL1, for which pyrimidine unpairings are structural elements essential for binding.

Thus, the main mechanism of action of the molecules known in the state of the art is inhibition of the sequestration exerted by hairpin loops with CUG repeats on MBNL proteins, thereby preventing the interaction between the hairpin loops and MBNL proteins, without exerting a direct effect on the destructuration of the hairpin loops.

On the contrary, the present invention focuses on the screening of drugs from chemical libraries in order to identify compounds with relevant biological activity to treat diseases the etiology whereof is based on the presence of toxic transcripts that comprise CUG or CCUG repeats, such as, for example: DM1, DM2 and SCA8. The compounds of the present invention are based on a more effective mechanism of action than those known in the state of the art, since, according to the most likely mechanism of action, the double-stranded hairpin loops composed of the toxic fragments with CUG repeats are bound and destructurated, or the RNA with the expansions remains in a single-chain configuration, thereby preventing the aberrant binding of MBNL1 and any other molecules the binding whereof might cause or intensify the pathological phenotype. Moreover, since they do not compete with the aberrant binding between MBNL1 and the CUG hairpin loops, which are structurally very similar to the protein's natural targets, they are not expected to interfere with the MBNL1-regulated transcripts in the cell. In the present invention, the *Drosophila* fly was used as a toxicity model for CTG repeats (Garcia-Lopez *et al.,* 2008), where the compounds of the invention were assayed and their mechanism of action on the toxicity of the CTG repeats was determined. Subsequently, their efficacy was validated in DM1 vertebrate models.

### DESCRIPTION OF THE INVENTION

In the present invention, we performed a process for identifying compounds with therapeutic potential to prevent or treat diseases the etiology whereof is based on the presence of toxic transcripts that comprise CUG, CCUG, CGG, CAG and AAG repeats, such as, for example: DM1, SCA8, DM2, FXTAS, HD and FA, which comprises the *in vivo* screening of hexapeptide chemical libraries, preferably in a *Drosophila* model of the disease, and the selection of those compounds that revert the pathological condition.

The size of the molecules to be used for the search of potentially therapeutic agents is an important aspect to be considered, since a molecular weight that is too high limits the cells' absorption of the compound. The optimisation of molecules to produce more active compounds is usually accompanied by an increase in the final size thereof. However, a molecular weight greater than 1000 reduces the molecules' therapeutic potential, by reducing the bioavailability thereof, which makes it necessary to start from small compounds. Thus, 80% of the drugs currently commercialised have a molecular weight of less than 450. The mean molecular weight of an amino acid is about 135 Da. Therefore, the approximate size of a hexapeptide varies around 810 Da. Screening peptide libraries formed by a number of amino acids greater than 6 would lead to excessively big molecules. On the other hand, although there are collections of di-, tri-, tetra- and pentapeptides, increasing the number of amino acids that form the molecule leads to a greater quantity of defined peptides during deconvolution, thereby increasing the probability of finding an active compound.

It was observed that the phenotype exhibited by the *Drosophila* model described in (Garcia-Lopez *et al.,* 2008), which expresses 480 CTG repeats (CTG(480)), leading to a lethality phenotype in the mature pupa stage, responds to levels of Mbl and is susceptible to chemical modification, which makes this model suitable for the systematic search of compounds with the therapeutic potential to prevent or treat diseases the etiology whereof is based on the presence of toxic transcripts that comprise CUG, CCUG, CGG, CAG and AAG repeats, such as, for example: DM1, SCA8, DM2, FXTAS, HD and FA.

The present invention begins with the screening of a combinatorial peptide chemical library in a positional scanning format. This chemical library was composed of 120 vials, each of which consisted of a mixture of hexapeptides that share a single amino acid in a specific position and differ in all the rest. Therefore, when a positive vial is detected, an active amino acid is identified in a given position. The combination of the most active amino acids in each of the positions assayed (vials) is known as deconvolution. In a preferred embodiment of the invention, in order to prolong the average life of the peptides in the body, the peptides from the chemical library used in the present invention were composed of the D-stereoisomers of natural amino acids, which are not recognised by intestinal proteases and are less susceptible to degradation.

Thus, the present invention relates to compounds that comprise hexapeptides with verified *in vivo* therapeutic potential in *Drosophila* and mice, for the prevention and/or treatment of diseases the etiology whereof is based on the presence of toxic transcripts that comprise CUG, CCUG, CGG, CAG or AAG repeats, such as, for example: DM1, SCA8, DM2, FXTAS, HD and FA. The mechanism of action of the compounds of the present invention is more effective than that exerted by the compounds known in the state of the art, since they are capable of binding to and destructurating the double-stranded hairpin loops formed by the toxic fragments with said repeats, or maintaining the RNA with the expansions in a single-strand conformation, thereby preventing the aberrant binding or sequestration of MBNL and any other molecules the binding whereof to CUG hairpin loops could also cause or intensify the pathological phenotype.

More specifically, in the present invention compounds were assayed which comprise peptides with Formula **(I) (A-B-C-D-E-F)** or pharmaceutically acceptable stereoisomers, mixtures, salts or mimetic peptides thereof. Below, we define the amino acids that may be a part of each of positions **A** to **F** of Formula I, naming said amino acids with both the three-letter code and the lower-case code that are conventionally used to represent the stereoisomers of natural amino acids:
- **A** may be amino acids cys (c) or pro (p),
- **B** may be amino acids pro (p) or gln (q),
- **C** is amino acid tyr (y),
- **D** may be amino acids ala (a) or thr (t),
- **E** may be amino acids gln (q) or trp (w); and
- **F** is amino acid glu (e).

In a preferred embodiment, the amino acids that are a part of the peptides of the invention are D-amino acids, which are not recognised by intestinal proteases and are less susceptible to degradation. However, as shown in the examples, peptides composed of L-amino acids are also active.

In the present invention, mimetic peptide is understood to mean a peptide organic molecule characterised by a complementary, homologous and/or equivalent sequence to that of the peptides with Formula (I). The compounds of the present invention also comprise functional complementary, homologous and/or equivalent sequences of the peptides with general Formula I. In a preferred embodiment, the compounds of the present invention comprise a homologous sequence which is at least 80% identical or homologous to the sequence of the peptide with general formula I. Moreover, the compounds of the present invention may comprise peptides that are chemical analogues of those with Formula I, derived cyclic peptides, dimers and/or multimers.

As demonstrated in the present invention, the compounds that comprise the aforementioned peptides may be used in the prevention and/or treatment of diseases the etiology whereof is based on the presence of toxic transcripts that comprise CUG, CCUG, CGG, CAG or AAG repeats, such as, for example: DM1, SCA8, DM2, FXTAS, HD and FA. The hexapeptides corresponding to sequences SEQ ID NO: 1 to 16 were tested for the treatment of said diseases, and hexapeptide p10 (SEQ ID NO: 10) was especially preferred, since it reverted toxicity in the aforementioned *Drosophila* model, both in the brain and the muscle, in a dose-dependent manner in both cases. Moreover, the endogenous expression of a peptide with a sequence that comprises p10, in its retro-inverse configuration, reverted phenotypes in the eye and the muscle of model flies, thereby confirming the effect of p10 and the derivatives thereof on the toxicity of CTG repeats. The results of the alanine scanning, as well as the generation of transgenic flies, demonstrated that all the amino acids in the sequence of p10 are necessary for the activity thereof and that the latter lies in the lateral chains of its residues. p10 binds to CUG repeats *in vitro,* deploying the hairpin loop. This bond is dependent on the peptide sequence and is greater when the number of repeats increases. The intramuscular injection of p10 in DM1 model mice reverted the splicing defects of the muscle transcripts, as well as defects at the histological level. This effect is systemic and was maintained for at least 4 weeks following a single injection.

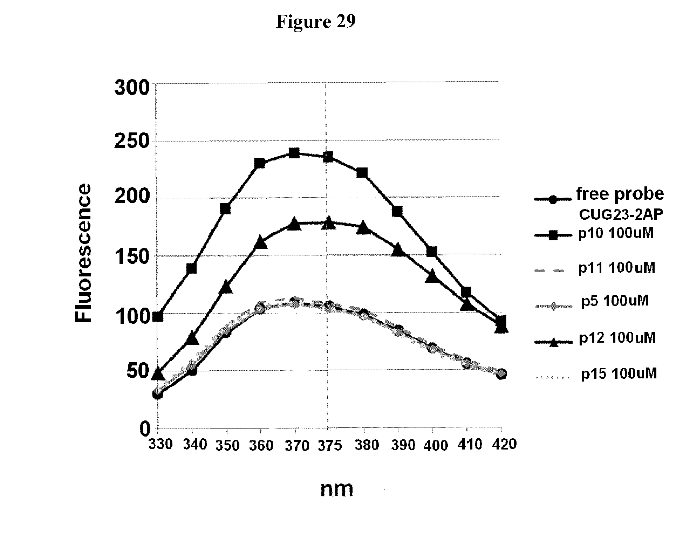

Although, as mentioned above, p10 is the preferred compound of the present invention, since it is the most effective of those tested, the present invention also demonstrates that other compounds included within Formula I, such as, for example, p11, p5, p12 and p15, also present different degrees of specificity and efficacy in the presence of toxic CUG transcripts (see Example 16 and Figure 29).

Therefore, a first aspect of the present invention relates to compounds that comprise the hexapeptides with Formula I, or mimetic peptides thereof, to be used in the prevention and/or treatment of diseases the etiology whereof is based on the presence of toxic transcripts that comprise CUG, CCUG, CGG, CAG and AAG repeats, such as, for example: DM1, SCA8, DM2, FXTAS, HD and FA.

A second aspect of the present invention relates to the use of said compounds for the preparation of a pharmaceutical composition designed for the prevention and/or treatment of diseases the etiology whereof is based on the presence of toxic transcripts that comprise CUG, CCUG, CGG, CAG and AAG repeats, such as, for example: DM1, SCA8, DM2 FXTAS, HD and FA.

The compounds disclosed in the present invention may be used as active principles in human patients or animals, and may be prepared in formulations and/or administered, in accordance with the knowledge in the state of the art of galenic development. Thus, a third aspect of the present invention relates to pharmaceutical compositions that comprise at least one of the peptides of the invention combined with at least another active principle. The composition could also comprise at least one excipient accompanying the active principle as an inactive substance, which, for example, contributes to the absorption of said active principle in the body or to the activation thereof.

Said excipients could be designed for the maintenance of the bound ingredients of the composition, such as, for example: starches, sugars or celluloses; fillers such as, for example: vegetable cellulose, dibasic calcium phosphate, safflower; disintegrating agents; lubricants, such as, for example: talc, silica or steroid fats; coating agents; sweetening agents; flavouring agents; colouring agents; etc.

Said composition may be administered by any administration route useful for the active principle to reach its therapeutic target, for example, by digestive route (oral, sublingual, gastroenteric or rectal), parenteral route (subcutaneous, intramuscular, intravenous, intra-arterial, intrarachidian, intraperitoneal, intradermal or intra-articular), respiratory route or topical route.

Another aspect of the present invention relates to a method for the prevention and/or treatment of diseases the etiology whereof is based on the presence of toxic transcripts that comprise CUG, CCUG, CGG, CAG and AAG repeats, such as, for example: DM1, SCA8, DM2, FXTAS, HD and FA, which comprises administering to a patient a therapeutically effective quantity of a pharmaceutical composition that comprises at least one of the hexapeptides of the invention. In the present invention, "therapeutically effective quantity" is understood to mean that quantity which is capable of preventing or treating the pathological condition associated with the diseases the etiology whereof is based on the presence of toxic transcripts that comprise CUG, CCUG, CGG, CAG and AAG repeats, such as, for example: DM1, SCA8, DM2 and SCA8.

In the present invention, positive results were observed at p10 concentrations greater than 40 µM and less than 250 µM, preferably 70.4 µM, in *a Drosophila* model for DM1.

### DESCRIPTION OF THE FIGURES

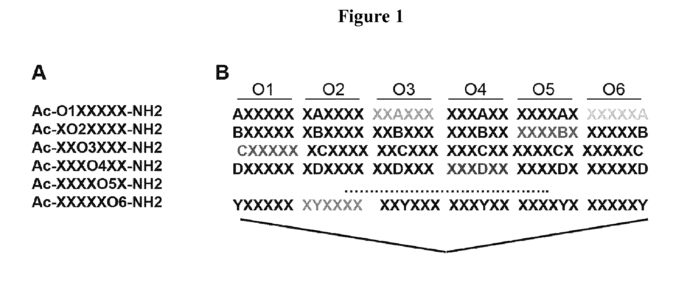

### Figure 1.- Positional scanning strategy.

(B) O1, O2... O6 represent defined positions occupied by the 20 D-stereoisomers of the possible natural amino acids and the X's refer to an equimolar mixture of 19 of the 20 D-stereoisomers of natural amino acids (cysteine is omitted in the X's, but not in the defined positions). The 6 positions for the 20 possible amino acids lead to 120 combinations, each of which represents a vial in the chemical library.

(A) Once the positive vials were identified, we studied which amino acid occupies the defined position in each. The combination of the positive amino acids to produce peptides of defined sequence is known as deconvolution.

Example 1 includes a detailed explanation of the conclusions obtained from Figure 1, as well as a more in-depth analysis thereof.

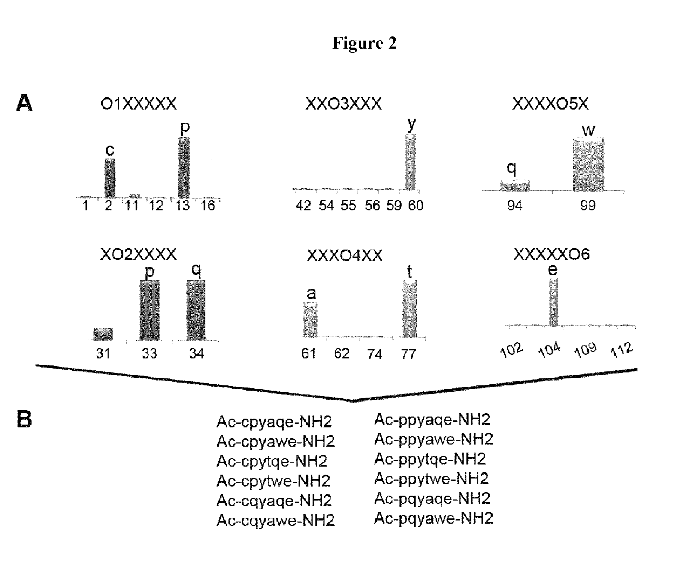

### Figure 2.- Result of the primary screening and deconvolution.

(A) The screening of the chemical library and the subsequent statistical analysis revealed a total of 28 positive vials (p-value < 0.05), the numerical code whereof is shown in the X-axis of each graph. Amongst these, the 10 amino acids indicated (corresponding to the vials with the greatest activity) were selected to perform the deconvolution. O1XXXXX, XO2XXXX, XXO3XXX, XXXO4XX, XXXXO5X and XXXXXO6 represent vials with defined amino acids for positions 1, 2, 3, 4, 5 and 6, respectively.

(B) The deconvolution led to 12 defined hexapeptides.

The Y-axis of the graphs shows the inverse p-values obtained in the primary screening as a measure of activity.

Example 1 includes a detailed explanation of the conclusions obtained from Figure 2, as well as a more in-depth analysis thereof.

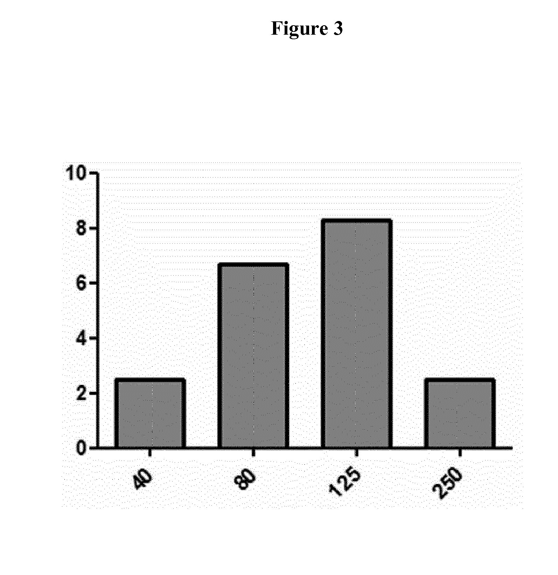

Figure 3.- Dose-response assay of p10 in a Drosophila model for DM. The Y-axis represents the fly models' increase in survival and the X-axis represents the p10 concentration (µM). p10 suppressed the lethality phenotype in *103Y-Gal4*/*+;UAS-CTG(480)*/+ flies most effectively between 80 and 125 µM. This trend was lost upon increasing the concentration to 250 µM. The increased survival value corresponds to ([treated born females]-[control born females]/n) x 100.

Example 1 includes a detailed explanation of the conclusions obtained from Figure 3, as well as a more in-depth analysis thereof.

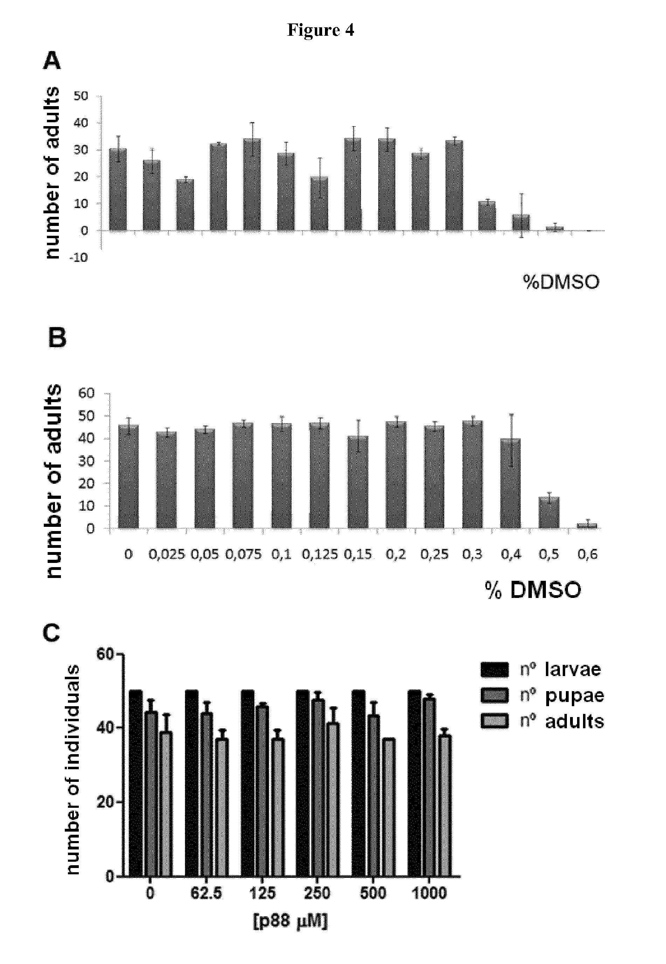

### Figure 4.- Study of the toxicity of p10 in wild individuals.

(A, B) This figure shows the dose-response behaviour to DMSO when the latter is administered to embryos (A) or L1 larvae (B) with the OrR genotype (wild genotype) in homemade food. The Y-axis represents the number of adults and the X-axis represents the % of DMSO.

(C) This figure demonstrates that p10 is not toxic in OrR individuals, since the number of L1 larvae that reach the pupa and adult stages was not different from the control at any concentration. Each assay was performed in triplicate with 50 individuals per replica (total of 150 individuals for each concentration). The Y-axis represents the number of individuals and the X-axis represents the p10 concentration (µM). In each group of three columns, the left-hand column represents the number of larvae, the middle column represents the number of pupae and the right-hand column represents the number of adults.

Example 2 includes a detailed explanation of the conclusions obtained from Figure 4, as well as a more in-depth analysis thereof.

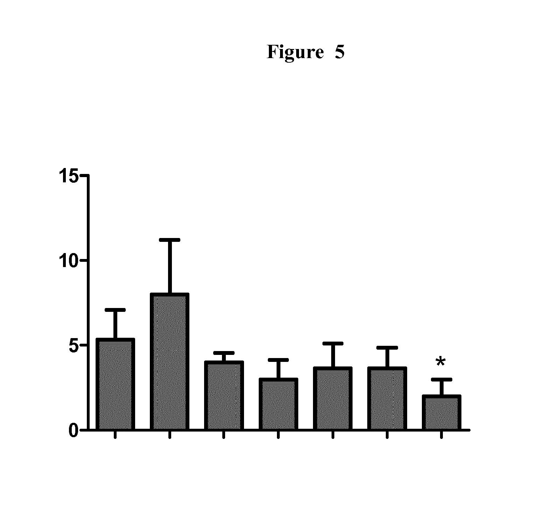

### Figure 5.- Alanine scanning.

This figure demonstrates that substituting any of the residues of peptide p10 with alanine causes a loss of activity. The peptides were administered to *103Y-Gal4*+/+*;UAS-CTG(480)*/+ larvae in the food. The Y-axis represents the number of females and the X-axis represents the hexapeptides of the invention assayed: the left-hand column corresponds to the control (0.1% DMSO) and the following, from left to right, to the p10 peptides and the peptides comprising alanine substitutions with the following sequences: ppyawa, ppyaae, ppaawe, payawe, apyawe. The bars show average values with the standard error thereof. * indicates a p-value < 0.05.

Example 3 includes a detailed explanation of the conclusions obtained from Figure 5, as well as a more in-depth analysis thereof.

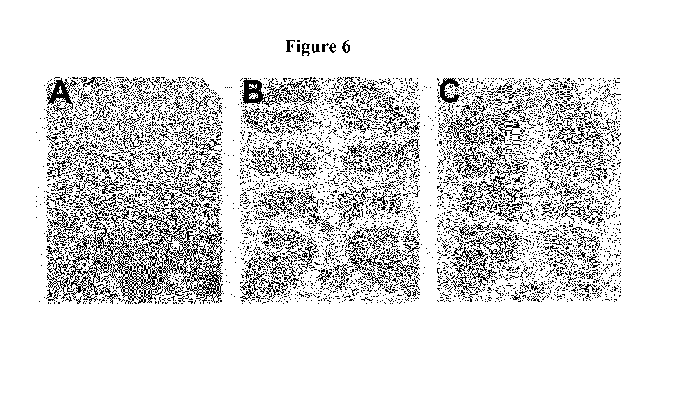

Figure 6.- Peptide p10 suppresses the toxicity of CTG(480) in the indirect flight muscles (IFMs).

The figure shows, from left to right, 1.5-µm cross-sections of the IFMs of normal flies (A), of flies that expressed CTG(480) (B) and of flies that expressed CTG(480) under the control of Mhc-Gal4 orally treated with peptide p10 (C). The flies treated with the peptide had larger muscular packages than the control with DMSO. The images were taken at a 10x magnification.

Example 4 includes a detailed explanation of the conclusions obtained from Figure 6, as well as a more in-depth analysis thereof.

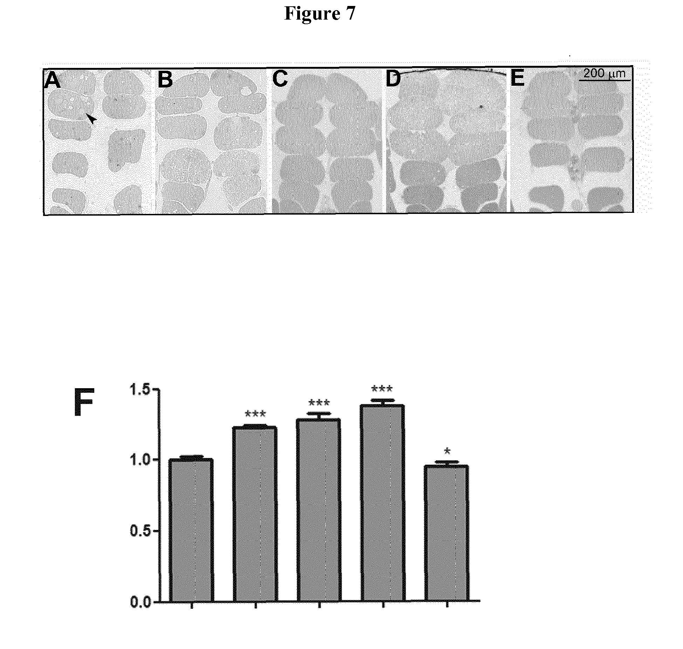

Figure 7.- Dose-response to peptide p10 in the IFMs.

Cross-sections of the IFMs of *Mhc-Gal4*/+*UAS-(CTG)480*/*+* flies treated with 0.12% DMSO (control) (A) and with peptide p10 at different concentrations: 62.5 µM (B), 125 µM (C), 250 µM (D) and 500 µM (E). The Y-axis of (F) shows the relative muscle area (µm). Moreover, (F) shows the results obtained, where the first column corresponds to DMSO, and the following, from left to right, correspond to p10 concentrations of: 62.5 µM, 125 µM, 250 µM and 500 µM. Between 62.5 µM and 250 µM, peptide p10 caused a significant increase in the muscle area as compared to the control flies (F), as well as a decrease in the loss of fibres (arrow head in A). At 500 µM, the area of the IFMs was smaller than in the control flies. The bars in the graph show average values with the standard error thereof. * indicates p-value < 0.05, *** indicates p-value < 0.0001.

Example 4 includes a detailed explanation of the conclusions obtained from Figure 7, as well as a more in-depth analysis thereof.

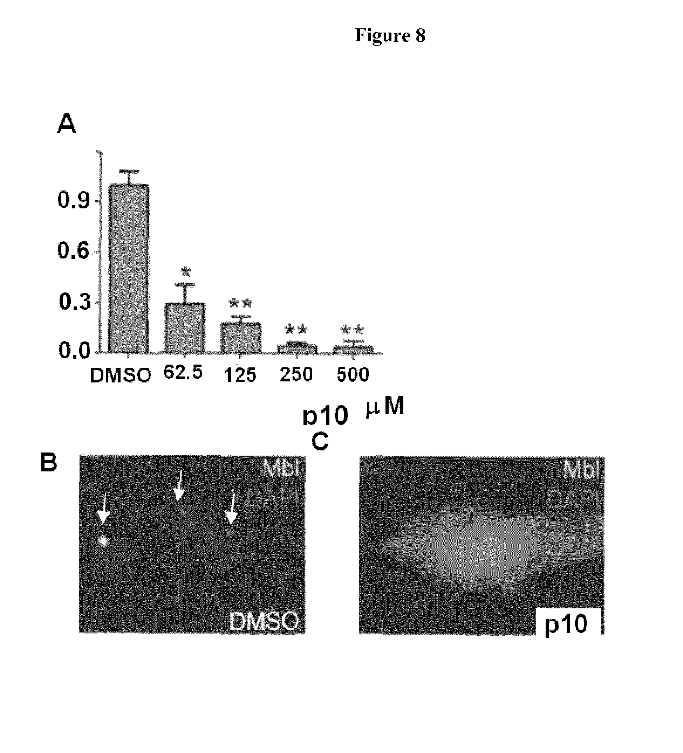

Figure 8.- p10 reduces the number of ribonuclear aggregates (foci) that comprise toxic CUG repeats in *Drosophila* and releases Mbl therefrom.

This figure shows that treatment with p10 drastically reduces the number of ribonuclear aggregates (foci) that comprise CUG repeats (Y-axis) at all the p10 concentrations tested (X-axis) (A). Moreover, it shows the detection of Mbl by immunofluorescence (grey) in the IFMs of flies treated with DMSO (0.12%; control) (B) or with p10 (250 µM) (C). The oral administration of p10 caused a change in the cellular distribution of Mbl, from a distribution in the form of aggregates (see arrows) (B), to a distribution in dispersed form (C). Staining of the nucleus was performed with DAPI. * means p < 0.05 and ** p < 0.0005. These results show a high level of efficacy in the reduction of two of the most evident molecular phenotypes characteristic of DM1 patients, following an *in vivo* treatment (in the Drosophila model) and oral ingestion of the compound (p10).

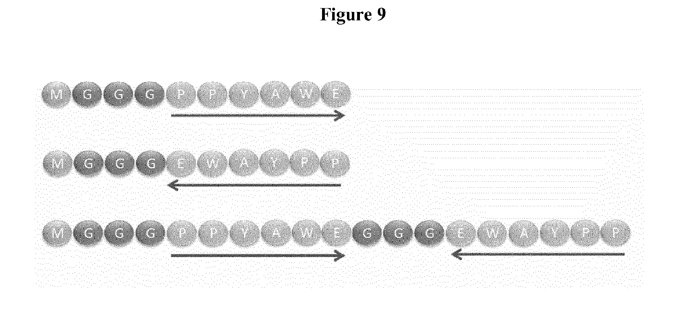

Figure 9.- Derivatives of peptide p10 designed for the endogenous expression thereof in *Drosophila.*

The sequence of the peptides of SEQ ID NO: 17-19 (p17-p19) is shown in a descending order. Shown are the initial Methionine, the three spacer Glycines and the direct (→) or retro-inverse (←) sequences of peptide p10.

Example 5 includes a detailed explanation of the conclusions obtained from Figure 9, as well as a more in-depth analysis thereof.

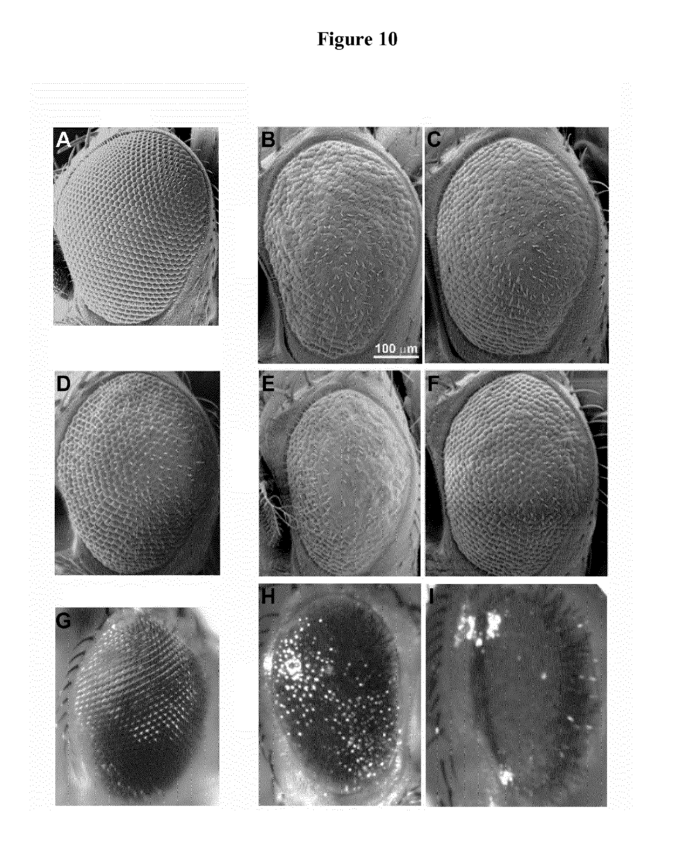

Figure 10.- The endogenous expression of an L-amino acid peptide that comprises p10 by means of the Gal4/UAS system suppresses the eye roughness caused by CTG(480).

UAS-p18 and p19 suppressed the toxicity of CTG(480) in the eye at 19°C and 21°C, leading to a less rough phenotype and a greater size of the eye than in the control flies (B, E vs C, F). Upon increasing the temperature, this phenomenon was inverted and UAS-p18 and p19 became enhancers of the effect of the repeats (25°C; H vs I). UAS-p18 by itself did not produce a rough eye at 25°C (G). Figure (A) shows a wild eye. (D) The interaction between CTG(480) and *MBNL1* was used as a positive control. Images (A-F) were taken under a scanning electron microscope. Images (G-I) were taken under the magnifying glass.

Example 5 includes a detailed explanation of the conclusions obtained from Figure 10, as well as a more in-depth analysis thereof.

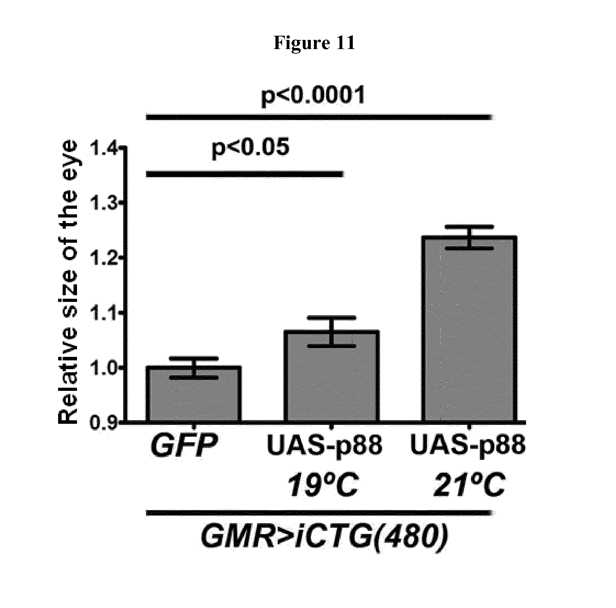

Figure 11.- Quantification of the suppression of an eye phenotype by the co-expression of (CTG)480 and different variants of the sequence of p10.

The quantification of the length of the eye in the dorso-ventral axis of *GMR-Gal4 UAS-CTG(480)*/*UAS-p19* flies showed a significant increase in the size of the eye in the control flies (*GMR-Gal4 UAS-CTG(480)*/*+;* p-value < 0.05, T-test). This effect was greater upon increasing the temperature from 19°C to 21°C. The Y-axis shows the relative size of the eye with respect to the control without peptide and the X-axis shows, from left to right, the control column, the column for UAS-p19 at 19°C and the column for UAS-p19 at 21°C.

Example 5 includes a detailed explanation of the conclusions obtained from Figure 11, as well as a more in-depth analysis thereof.

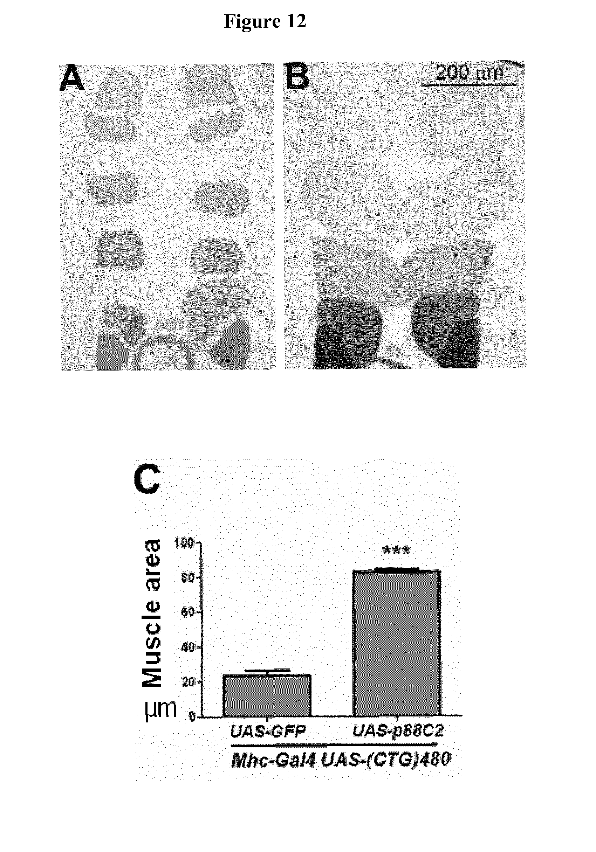

Figure 12. Endogenous expression of an L-amino acid peptide that comprises p10 in the IFMs by means of the Gal4/UAS system. Cross-sections of the thorax (1.5 µm) of flies that express CTG(480) (A) or CTG(480) and p18 simultaneously (B) under the control of the *Mhc* promoter. The presence of p18 caused an increase in the size of the muscles (C). Figure C shows the muscle area in the Y-axis, and the X-axis represents the effect of UAS-GFP, as a negative control, in the left-hand column and the effect of UAS-p18 in the right-hand column.

Example 5 includes a detailed explanation of the conclusions obtained from Figure 12, as well as a more in-depth analysis thereof.

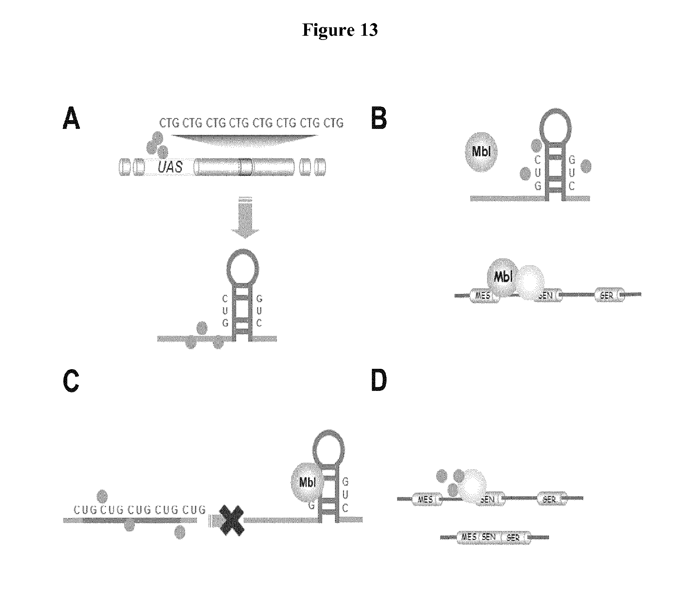

Figure 13.- Possible mechanisms of action of peptide p10 on the toxicity of CTG(480).

p10 could affect the expression of the repeats (A), shift Mbl from the RNA hairpin loops (B), destabilise the toxic hairpin loops (C) or act downstream from the repeats (D). However, as shown in the figures and examples below, the most probable mechanism of action of p10 is based on the destabilisation of the toxic hairpin loops (C).

Example 6 includes a detailed explanation of the conclusions obtained from Figure 13, as well as a more in-depth analysis thereof.

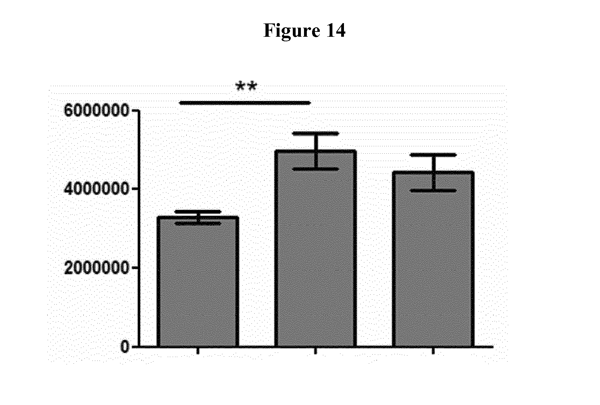

Figure 14. Effect of the expression of p10 on the expression of the luciferase reporter protein.

Treatment with p10 does not significantly affect the expression of luciferase induced by the Gal4/UAS system as compared to flies exposed to the same quantity of DMSO (0.12%). However, DMSO by itself affected the expression of the transgene. The bars show average values and the standard error thereof. ** indicates p-value < 0.01. The Y-axis shows luciferase (CPS) and the X-axis shows, from left to right, the effects of 0% DMSO, 0.1% DMSO and 250 µM of p10.

Example 7 includes a detailed explanation of the conclusions obtained from Figure 14, as well as a more in-depth analysis thereof.

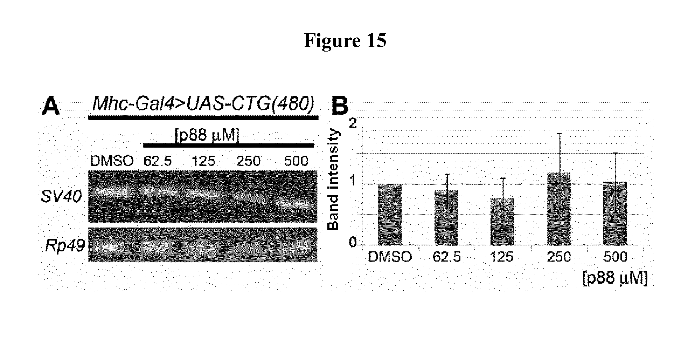

### Figure 15.- Effect of p10 on the expression of CTG(480).

(A) Agarose gel that shows the levels of expression of CUG(480) transcripts in *Mhc-Gal4*/+; *UAS-CTG(480)*/+ flies treated with 0.12% DMSO (control) (left-hand column) or with p10 at different concentrations (second column: 62.5 µM, third column: 125 µM, fourth column: 250 µM, fifth column: 500 µM) amplified by means of semi-quantitative RT-PCR of a region of the *SV40* terminator. The *Rp49* gene transcripts were used as a cDNA template loading control. The quantification of the intensity of the bands with the ImageJ programme did not reveal significant changes in any case (α = 0.05, T-test).

(B). The bars show averages and the standard deviation of the results obtained in (A). DMSO (control) (left-hand column) or p10 administered at different concentrations (second column: 62.5 µM, third column: 125 µM, fourth column: 250 µM, fifth column: 500 µM).

Example 7 includes a detailed explanation of the conclusions obtained from Figure 15, as well as a more in-depth analysis thereof.

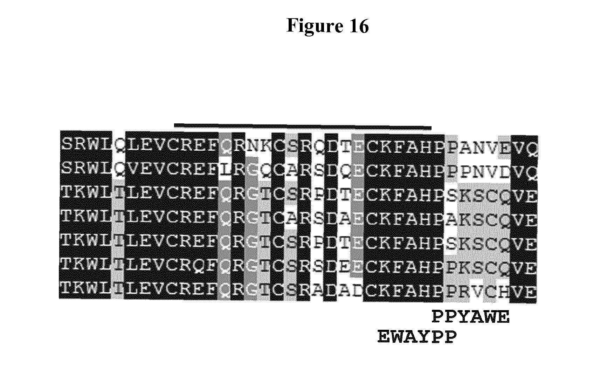

### Figure 16.- p10 partially aligns with the first zinc finger of Muscleblind.

In a descending order, the following are shown: the sequences of the first zinc finger of Muscleblind corresponding to *Drosophila melanogaster* (first row), *Caenorhabditis elegans* (second row), *Gallus gallus* (third row), *Danio Rerio* (fourth row) *and Homo Sapiens* (fifth, sixth and seventh rows: three human paralogues). The reverse sequence of p10 (last row) aligns with a critical region for binding of the protein to RNA. Conserved amino acids are shown in black.

Example 8 includes a detailed explanation of the conclusions obtained from Figure 16, as well as a more in-depth analysis thereof.

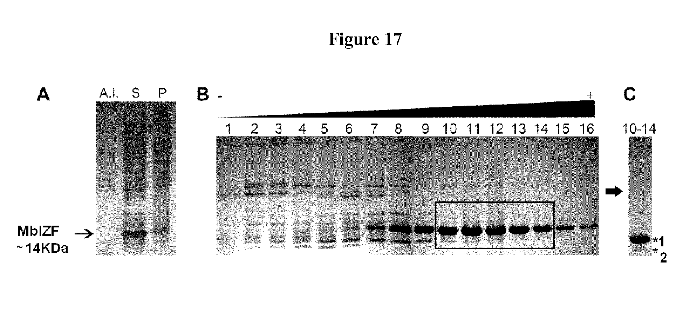

Figure 17.- Obtainment of the MblZF (Mbl zinc finger) protein in *E*. *coli.* (A) Expression of MblZF in strain BL21(DE3) of *E.coli.* A.I.: prior to induction with IPTG; S: supernatant of the cell lysates (soluble fraction); P: pellet of the cell lysates (insoluble fraction). Most of the protein is found in the soluble fraction (S), as shown in the arrow.
(B) Example of purification of the MblZF protein in imidazole gradient by FPLC. Fractions 10 to 14 were added to obtain a protein stock (C). Asterisks *1 and *2 show bands sequenced by mass spectrometry in order to confirm the identity of MblZF (*1). Band *2 turned out to be the 50S protein of *E.coli,* thereby ruling out the possibility that it was a degradation product of MblZF.

Example 9 includes a detailed explanation of the conclusions obtained from Figure 17, as well as a more in-depth analysis thereof.

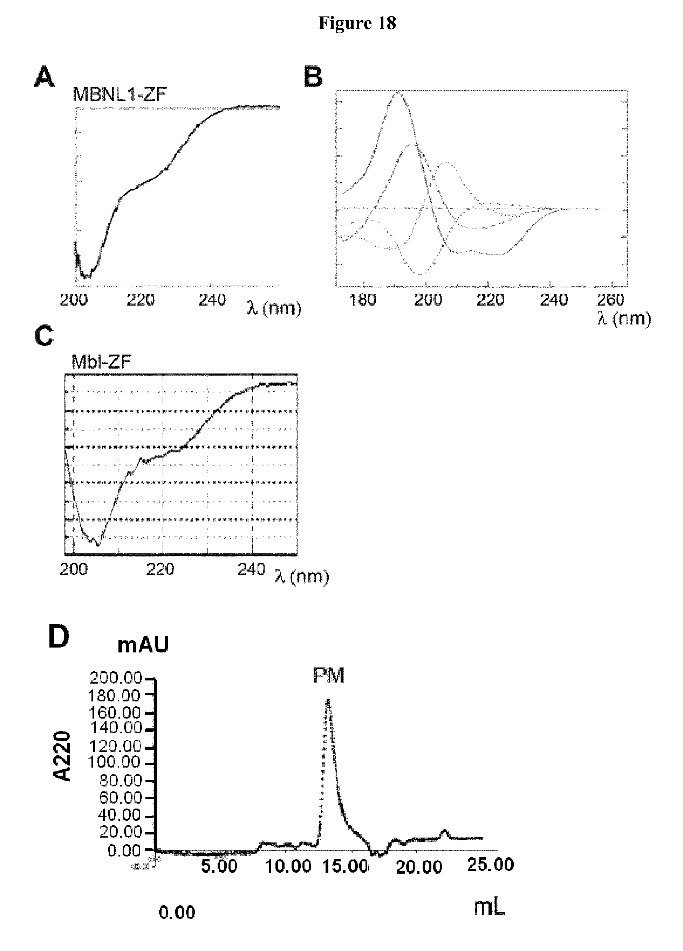

Figure 18.- CD (Circular Dichroism) Spectrum of the zinc fingers of Muscleblind proteins.

(A) The human MBNL1 protein (2 µM) presents a pronounced peak at 203 nm, and a weak peak at 220 nm, which indicates that MBNL1ZF (first pair of zinc fingers) does not have a secondary structure except for a small portion in the form of an alpha helix (compare with the pattern in B). MblZF behaved in a similar manner, with a pronounced peak at 205 nm and a weak peak at 222 nm (C). (D) MblZF eluted as a single peak when passing through a molecular exclusion column. The elution volume, compared to a pattern of molecular weight markers, coincides with the size of MblZF in monomer form.

Example 9 includes a detailed explanation of the conclusions obtained from Figure 18, as well as a more in-depth analysis thereof.

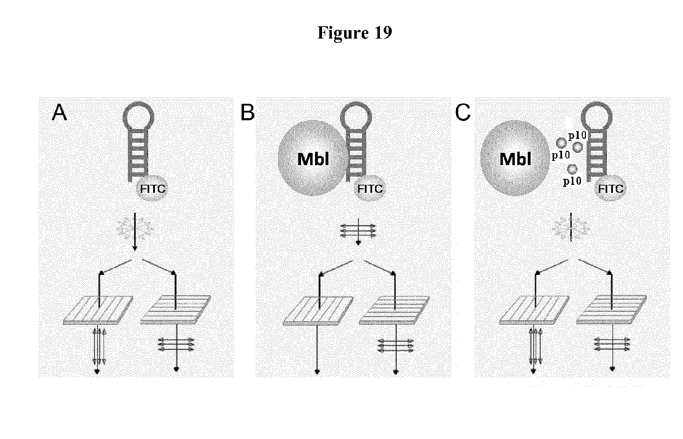

Figure 19.- Diagram of a fluorescence polarisation assay.

Fluorescence polarisation assays consist of labelling a small molecule with a fluorophore (CUG repeats conjugated with carboxyfluorescein in the case of the present invention; A), such that, when the latter binds to a molecule with a greater molecular weight (such as the MblZF protein), its rotational velocity is modified (B). Changes in the rotational velocity may be detected by exciting the molecule with polarised light beams in the vertical and horizontal planes, and measuring the polarisation direction of the fluorescence emitted. If a small-size molecule (p10) were capable of inhibiting the binding between MblZF and FAM-CUG23 (23 CUG repeats conjugated with the carboxyfluorescein fluorophore), the fluorescent RNA would once again increase its rotational velocity, reducing its polarisation (C).

Example 10 includes a detailed explanation of the conclusions obtained from Figure 19, as well as a more in-depth analysis thereof.

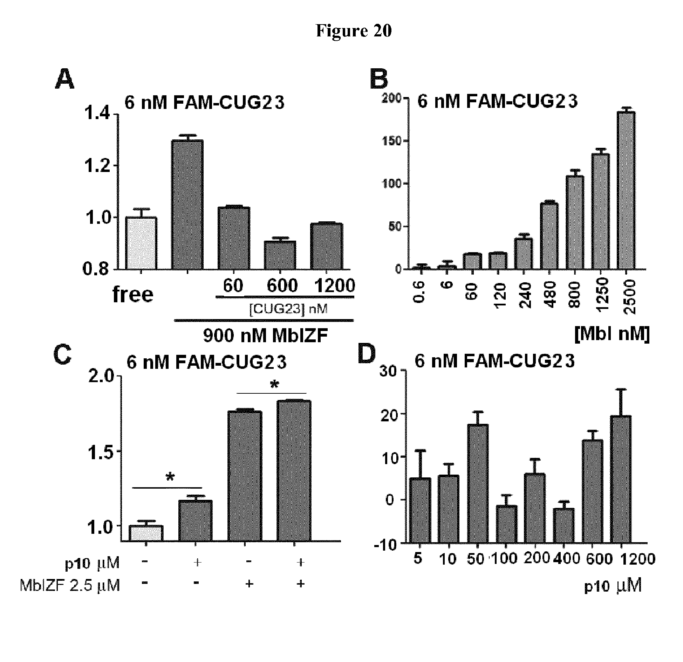

Figure 20.- MblZF and p10 bind to CUG repeats.

MblZF caused an increase in the polarisation of FAM-CUG23, produced by the binding between both molecules. This binding is reversible if it competes with an unlabelled CUG23 RNA (A) and is proportional to the quantity of MblZF (B). p10 also causes an increase in the polarisation of FAM-CUG23 (C). This increase is discreet due to the small size of the peptide and does not significantly change upon increasing the concentration thereof (D). p10 does not revert the effect of MblZF on FAM-(CUG)23. * indicates p-value < 0.05 (T-test). The Y-axis of figures A and C shows the relative polarisation and the Y-axis of figures B and D shows the increase in polarisation.

Example 10 includes a detailed explanation of the conclusions obtained from Figure 20, as well as a more in-depth analysis thereof.

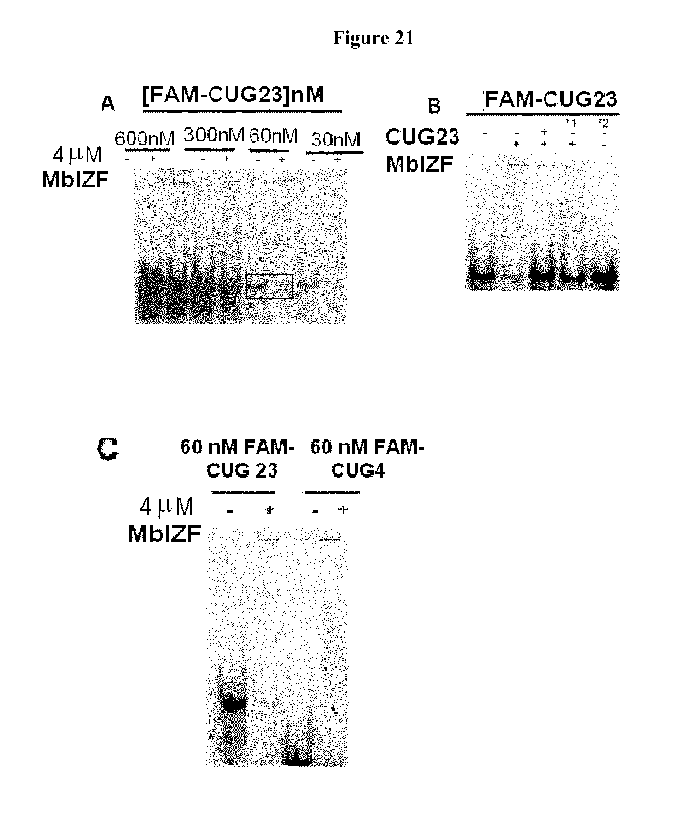

Figure 21.- Study of the binding of MblZF to FAM-(CUG)23 by means of gel retardation assays.

MblZF binds to FAM-CUG23, causing retention of the complex in the well. This occurs at all the RNA concentrations assayed for a fixed protein concentration (A). The binding between FAM-(CUG)23 and MblZF is specific, since it can compete with an unlabelled CUG23 RNA (FAM-(CUG)23:CUG23 proportion 1:100; Fig. B, lane 3), and this does not occur if the protein is denatured by heat prior to incubating with FAM-(CUG)23 (Figure B, lane 4). The collagen alpha-3 protein used at the same concentration as MblZF does not bind to RNA (Figure B, lane 5) (*1: MblZF denatured by heat, *2: incubated with collagen alpha-3). MblZF also binds to smaller-size RNAs (FAM-(CUG)4), to form complexes that are retained in the gel well (C).

Example 10 includes a detailed explanation of the conclusions obtained from Figure 21, as well as a more in-depth analysis thereof.

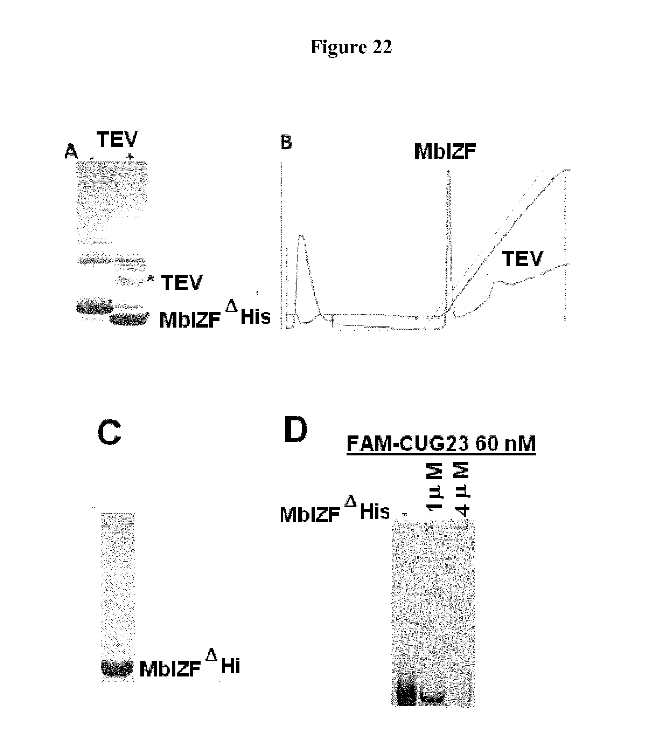

Figure 22.- The Histidine tail of MblZF does not affect its binding to FAM-(CUG)23.

(A) Gel that shows digestion with TEV protease to eliminate the 6 Histidines of the MblZF protein. The digestion product (MblZF^{ΔHis}) was passed through a Nickel affinity column in order to retain the Histidines and eliminate TEV, since the latter elutes differently than MblZF^{Δhis} (B). (C) Result following purification. (D) MblZF^{Δhis} binds to FAM-(CUG)23 and this binding is proportional to the protein concentration.

Example 10 includes a detailed explanation of the conclusions obtained from Figure 22, as well as a more in-depth analysis thereof.

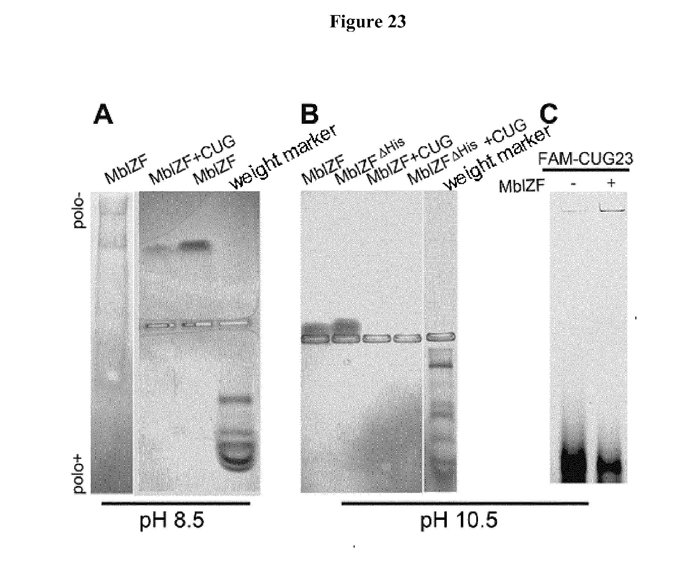

Figure 23.- The MbIZF/FAM-(CUG)23 complex does not migrate towards the positive pole.

(A) Under the electrophoresis conditions used, the MblZF protein barely entered into the gel (silver staining, Figure A left). If both the protein and the RNA-protein complex are run in a horizontal gel with the wells placed at the centre, only protein migrating towards the negative pole was observed. If the pH of the electrophoresis buffer is increased one point above the Ip of MblZF, the protein continued to migrate towards the negative pole (B). The MblZF^{Δhis} protein also did not enter into the gel (B). Fixating the RNA-protein complex by cross-linking with FA and resolving it in a denaturing gel (wherein SDS confers a negative charge to the protein) did not change the behaviour of MblZF (C).

Example 10 includes a detailed explanation of the conclusions obtained from Figure 23, as well as a more in-depth analysis thereof.

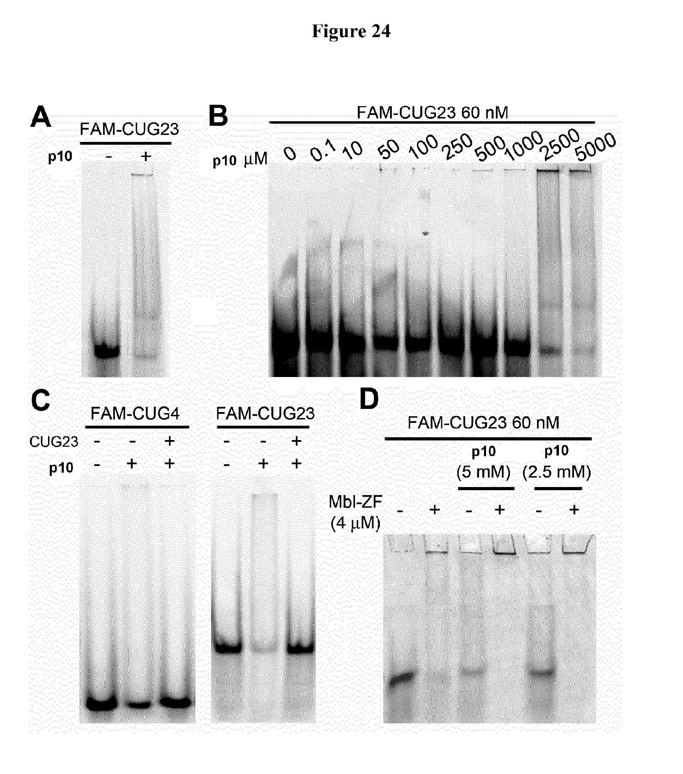

Figure 24.- p10 binds to CUG repeats without shifting MblZF.

(A) p10 (1 mM) may bind to FAM-(CUG)23 RNA (60 nM). (B) This binding is proportional to the quantity of peptide and is detected from ∼500 µM. p10 (1 mM) binds to short-size repeats (FAM-(CUG)4, 60 nM) with a lower affinity (C). Binding of the peptide to FAM-(CUG)23 does not interfere with the interaction of the MblZF protein (D), at least not significantly in this assay. Example 10 includes a detailed explanation of the conclusions obtained from Figure 24, as well as a more in-depth analysis thereof.

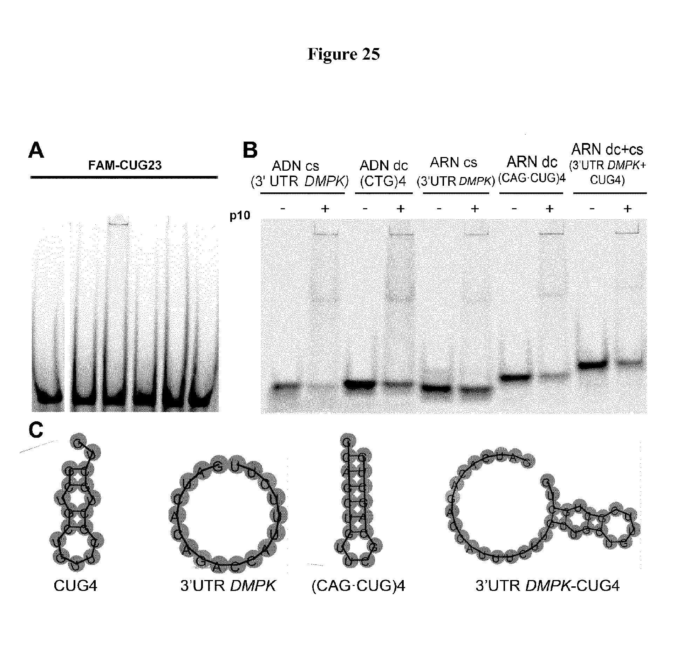

Figure 25.- Specificity of peptide p10.

(A) None of the five peptides of the alanine scanning (the first tube on the left is the control and the following five correspond to the peptides: ppyawa, ppyaae, ppaawe, payawe and apyawe) (2.5 mM) significantly bound to the FAM-(CUG)23 RNA (60 nM), thereby demonstrating that the interaction described for p10 is specific. (B) p10 (1 mM) may bind to both double-stranded (ds) and single-stranded (ss) RNA and DNA (60 nM). (C) Diagram that shows the secondary structure of the nucleic acids used in the experiment shown in (B).

Example 11 includes a detailed explanation of the conclusions obtained from Figure 25, as well as a more in-depth analysis thereof.

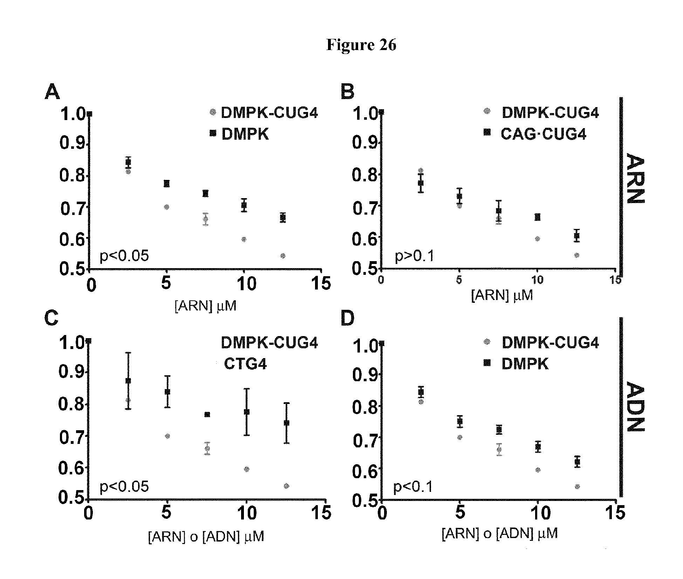

Figure 26.- p10 binds to DMPK-CUG4 with a higher affinity.

(A) Fluorescence extinction experiment of the tryptophan of p10 (5 µM). The peptide was incubated with different nucleic acids (2.5 µM, 5 µM, 7.5 µM, 10 µM and 12.5 µM). The fluorescence extinction rate (the Y-axis represents the relative fluorescence) was measured as the slope of the straight lines obtained upon representing the fluorescence emission values at 351 nm in relation to the free peptide for each concentration point. At least two measurements were performed for each point. In all cases, said rate was greater for DMPK-CUG4, albeit not significantly for CAG-CUG4 (B).

Example 11 includes a detailed explanation of the conclusions obtained from Figure 26, as well as a more in-depth analysis thereof.

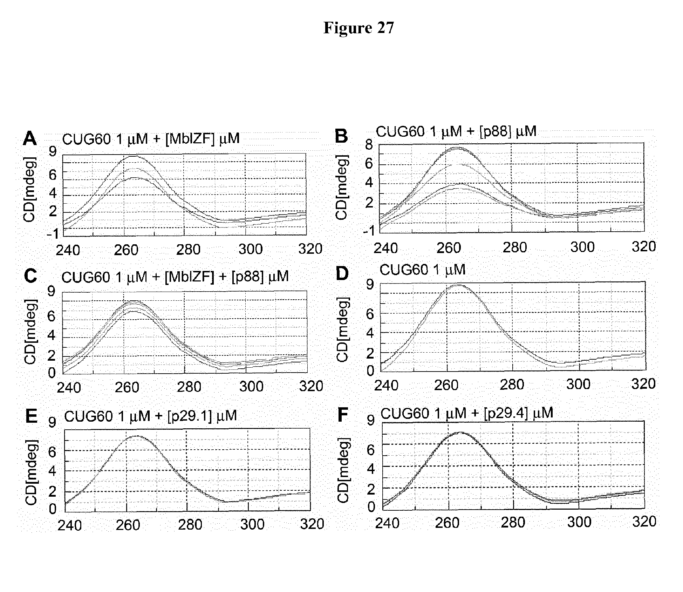

Figure 27.- p10 reduces the packaging of the CUG repeats.

Both MblZF (A, 1 µM and 1.5 µM) and p10 (B, 0.1 µM, 0.5 µM, 1 µM, 10 µM and 20 µM) changed the circular dichroism (CD) spectrum of CUG(60) (1 µM), reducing the emission peak of RNA to ∼265 nm proportional to the protein or peptide concentration. However, this effect was not reverted when the RNA was incubated jointly with MblZF and p10, regardless of the order of addition (C). This change was not caused by degradation of the RNA whilst the experiment lasted (D) and also did not occur when CUG60 was incubated with the alanine scanning hexapeptides with sequences ppyawa (E, 0.5 µM and 1 µM) and payawe (F, 1 µM).

Example 12 includes a detailed explanation of the conclusions obtained from Figure 27, as well as a more in-depth analysis thereof.

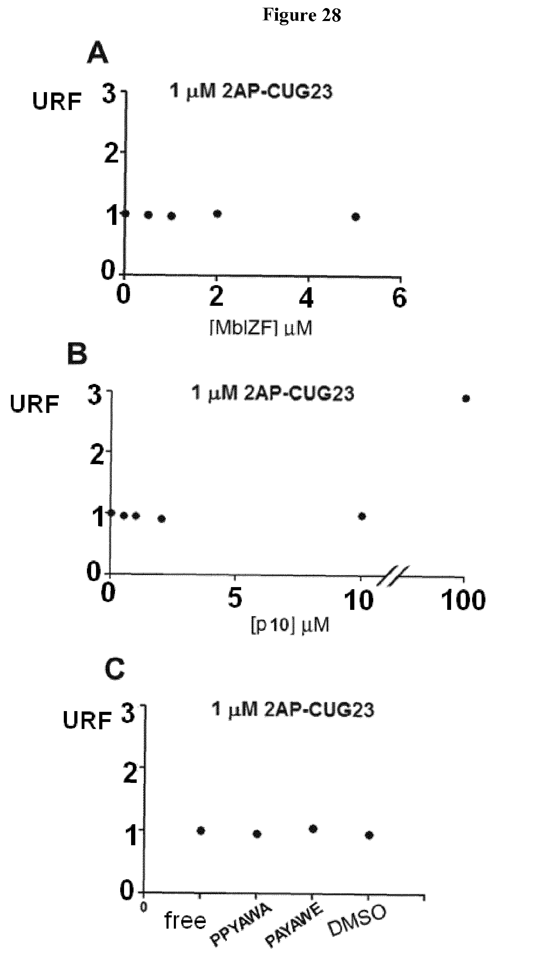

Figure 28.- p10 opens the hairpin loops formed by the CUG repeats.

Measurements of the fluorescence emitted (Y-axis), measured as relative fluorescence units (RFU), by 2-aminopurine in a (CUG)23 RNA (1 µM) in the presence of MblZF (0.1 µM, 1 µM, 2 µM and 5 µM) (A) or p10 (0.1 µM, 1 µM, 2 µM, 5 µM and 100 µM) (B), relative to the fluorescence emitted by the free RNA. p10 caused a 2.9-increase in the fluorescence of 2-AP at 100 µM, which indicates a change towards single-stranded. This effect was not observed when incubating the RNA with DMSO, or with the alanine scanning peptides with sequences: ppyawa and payawe (C).

Example 12 includes a detailed explanation of the conclusions obtained from Figure 28, as well as a more in-depth analysis thereof.

Figure 29.- Comparative assay of several peptides of the invention (p10, p11, p5, p12 and p15) in the destructuration or opening of the hairpin loops formed by the CUG repeats.

This figure illustrates an experiment which uses the 2-aminopurine assay (as in the preceding figure) to assay 4 peptides (p11, p5, p12 and p15) in addition to p10, obtained from the deconvolution of the combinatorial peptide library. The results indicate a correlation between the activity obtained with the peptides (Table 1) and the capacity of the CUG23 probe to destructurate secondary structures and become single-stranded. The concentration used in all cases is 100 µM (1:100 proportion of RNA vs. p10), where p10 had shown a positive response (preceding figure). Therefore, this experiment demonstrates that, although p10 is the preferred peptide, since it is the most active, the rest of the peptides of Table 1, particularly p11, p5, p12 and p15, also present appreciable levels of specificity and efficacy.

Example 16 includes a detailed explanation of the conclusions obtained from Figure 29, as well as a more in-depth analysis thereof.

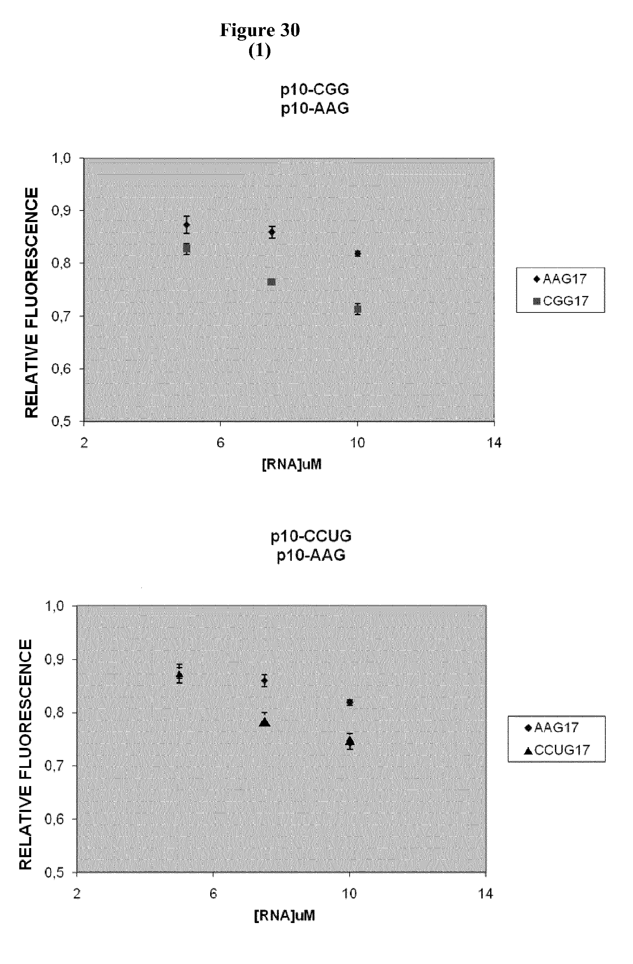

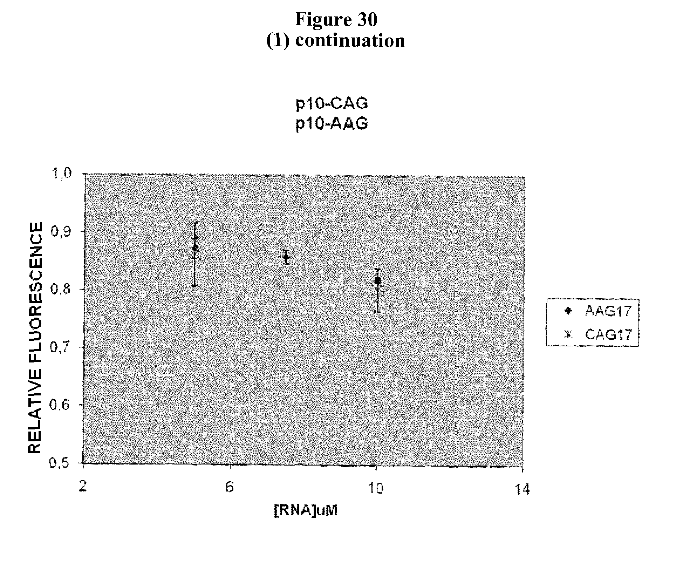

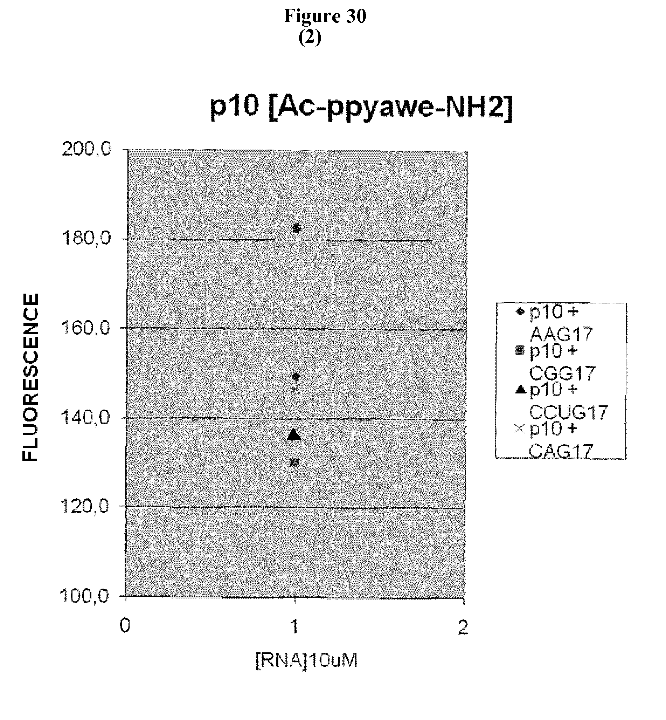

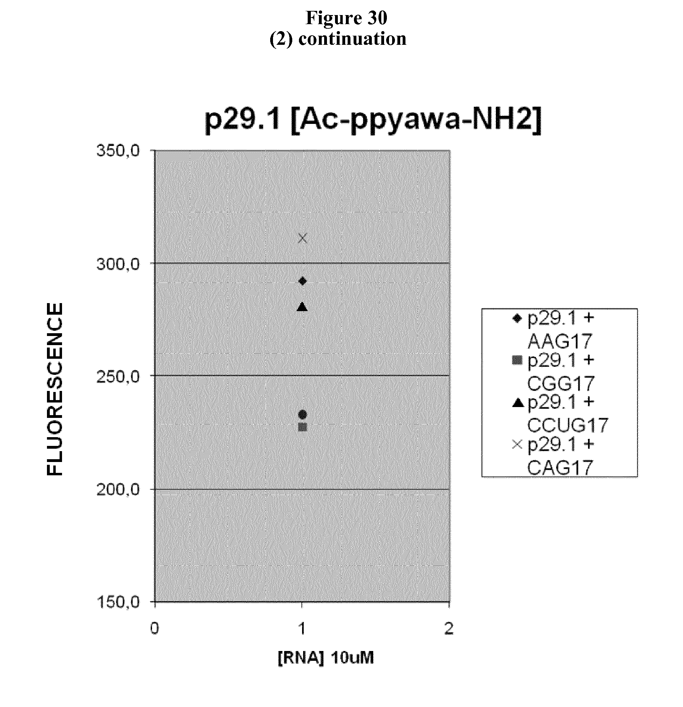

Figure 30.- Assay of the peptides of the invention (particularly p10) in the binding to hairpin loops formed by repeats different from CUG repeats, such as, for example, AAG, CGG, CCUG and CAG.

This figure illustrates an experiment which tested p10 and its capacity to bind to repeats different from CUG repeats, but which are also involved in diseases where the toxicity of RNAs with repeat expansions has been described. The tryptophan extinction assay was used, using (1) p10 + AAG, CGG, CCUG and CAG at different RNA concentrations (5 µM, 7.5 µM and 10 µM). AAG is used as a reference repeat (control) because it has been disclosed that it does not form secondary structures. On the other hand, (2) shows the same experiment, but using one of the peptides obtained following the alanine scanning (ppyawa) and showing the results at the highest RNA concentration (10 µM) used.

Example 16 includes a detailed explanation of the conclusions obtained from Figure 30, as well as a more in-depth analysis thereof.

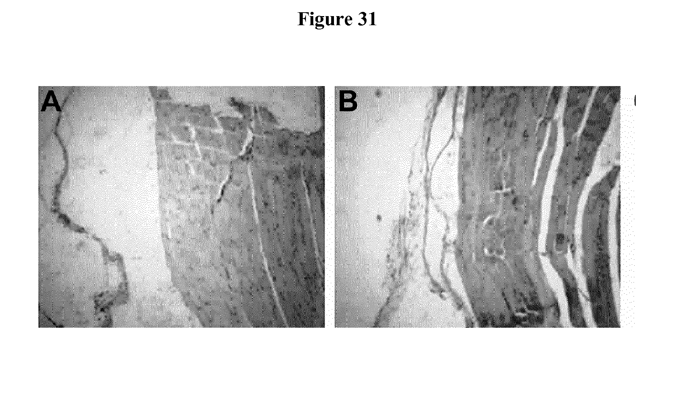

Figure 31.- Example of histopathological analysis.

The intramuscular injection of 0.2% DMSO (A) or 0.5 µg of p10 (B) in *FVB* mice generated a small mixedematous area, with barely any relevant associated inflammatory reaction in both cases.

Example 13 includes a detailed explanation of the conclusions obtained from Figure 31, as well as a more in-depth analysis thereof.

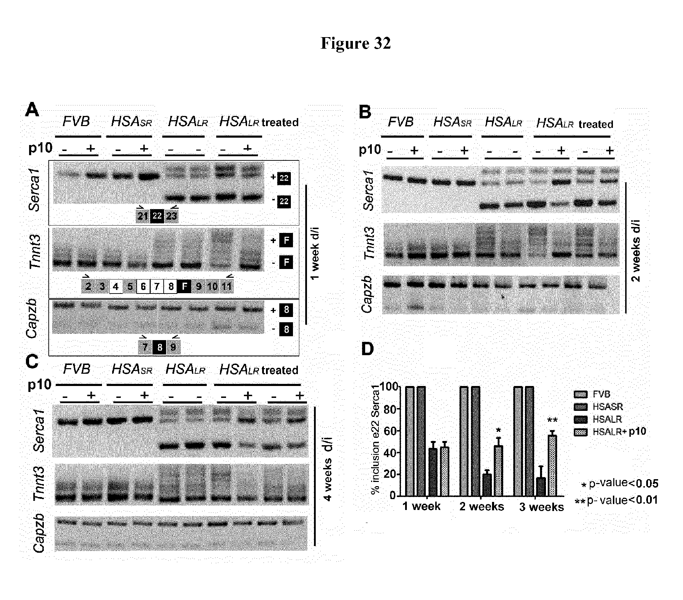

Figure 32.- p10 reverts the splicing defects of *Serca1* and *Tnnt3* in DM1 model mice.

The intramuscular injection of 2% DMSO (-) or 10 µg of p10 (+) increased the inclusion percentage of exon 22 of the *Serca1* transcripts 2 and 4 weeks p/i (B, C and D) and the exclusion percentage of foetal exon F of *Tnnt3* 1, 2 and 4 weeks after the injection (p/i; A, B and C). Peptide p10 did not alter the processing of these transcripts in *FVB* or *HSA^{SR}* mice, and neither did it affect the control Capzb transcripts (A-C). Figures (A-C) show the results of RT-PCR performed for 25 cycles. The horizontal lines join the left extremity (injected with serum with 2% DMSO) and the right extremity (injected with 10 µg of peptide) of the same animal, in that order. The bars in (D) correspond to average values with the standard error thereof. The p-values were determined using a T-test.

Example 14 includes a detailed explanation of the conclusions obtained from Figure 32, as well as a more in-depth analysis thereof.

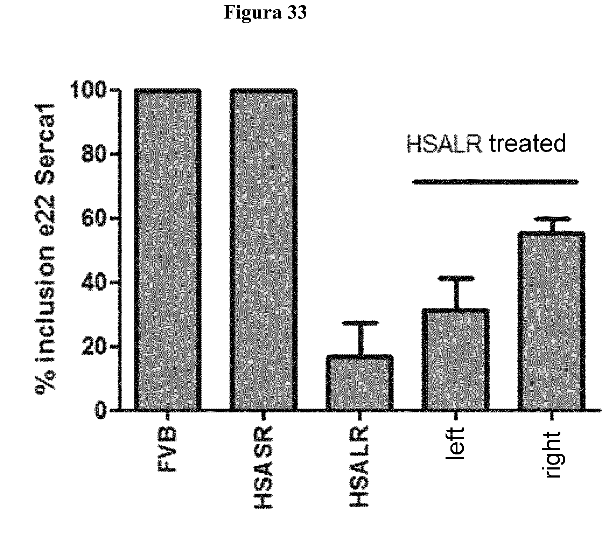

Figure 33.- Systemic effect of p10 on the alternative splicing of *Serca1.* The inclusion percentage of exon 22 (Y-axis) in *FVB* and *HSA^{SR}* mice 4 weeks after the injection of 10 µg of p10 was 100%. This value was 16.8% ± 10.5 in the *HSA^{LR}* animals injected with 2% DMSO in both legs. In the *HSA^{LR}* animals treated, the inclusion percentage turned out to be 55.1% ± 4.9 in the leg injected with p10 and 31.5% ± 9.8 in the left leg of the same animal (injected with serum with 2% DMSO).

Example 14 includes a detailed explanation of the conclusions obtained from Figure 33, as well as a more in-depth analysis thereof.

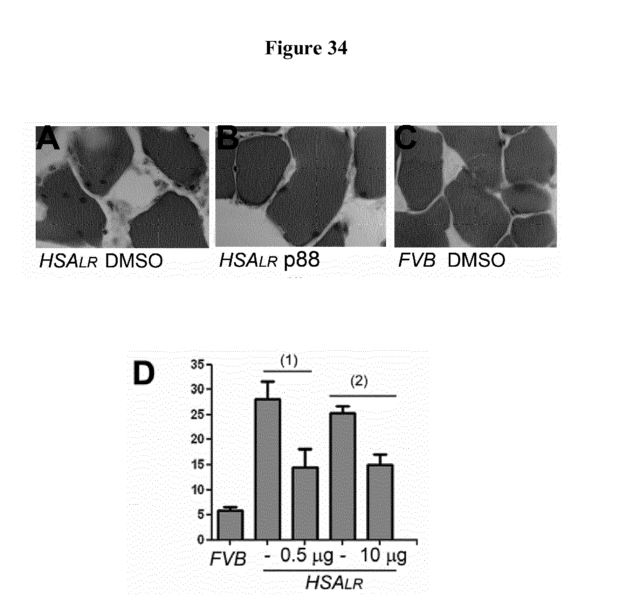

Figure 34.- p10 reduces the number of muscle cells with a central nucleus.

Cryosections of the anterior tibial muscle stained with hematoxylin eosin showing the presence of central nuclei in the *HSA^{LR}* animals (A), whereas they are placed in the periphery of the cells in the *FVB* animals (C). p10 significantly reduced the percentage of fibres with a central nucleus in *HSA^{LR}* mice after 4 weeks had elapsed since the injection of both 0.5 µg and 10 µg (B and D). (1) Indicates animals the control whereof was injected with 0.2% DMSO. (2) Indicates animals the control whereof was injected with 2% DMSO.

Example 15 includes a detailed explanation of the conclusions obtained from Figure 34, as well as a more in-depth analysis thereof.

Below we show the examples performed in the present invention in order to illustrate the results achieved, which are not intended to limit the scope of the invention.

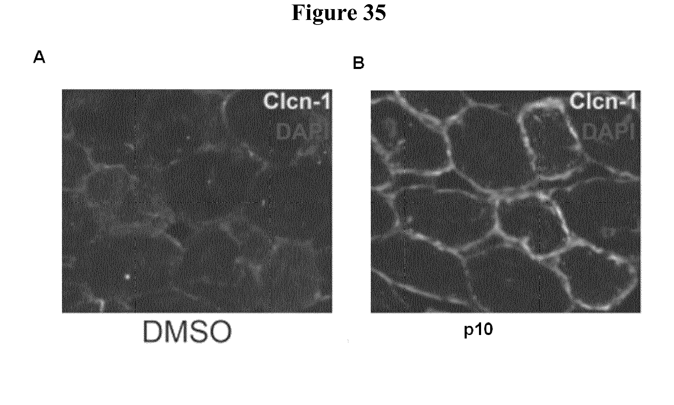

Figure 35.- p10 suppresses histological defects at the muscular level in DM1 model mice.

This figure represents an experiment performed in the mouse model for the disease, where the location of the Clcn-1 protein is detected in skeletal muscle sections. Clcn-1 is one the genes altered at the splicing level in the musculature of patients with DM1 (A). It is observed that the administration of p10 leads to the restoration of normal levels of protein at the membrane level (B). The intramuscular injection of p10 was performed at the muscular level in the hind extremities of the mice. This result expands the capacity (already observed in Figures 32-34) of a treatment with p10 in an *in vivo* DM1 model to improve cellular and functional aspects that are expressed in an aberrant manner in this human pathology.

### EXAMPLES

### Example 1. Screening of a combinatorial hexapeptide chemical library

Each chemical library vial is composed of a mixture of 2.5 million peptides that share a single amino acid in a defined position and differ in the rest (Figure 1A). Thus, the combination of the 6 positions defined by the 20 amino acids that may occupy them leads to the 120 vials that make up the chemical library, adding to a total of 50 million different sequences. The use of positional scanning combinatorial chemical libraries is based on the principle that each position of the molecule is assayed independently from the rest, such that it is possible to define the best candidate amino acid for each of the six hexapeptide positions. Thus, by reading of the results of the screening, the most active amino acids for each position were obtained. Subsequently, on the basis of their combinations, peptides of defined sequence were synthesised (what is known as deconvolution) (Figure 1B) and these molecules were assayed again.

The 120 vials that make up the chemical library were individually assayed on the lethality phenotype in pupae caused by the expression of 480 CTG under the control of 103Y-Gal4 at a concentration of 80 µM. Statistical analysis of the results revealed a total of 28 positive vials (p < 0.05; 23.3% of the total), each of which represents an active amino acid in a specific position. Thus, for positions O1 02, 03, 04, 05 and 06 of the hexapeptide, a total of six, three, six, four, two and seven active amino acids were obtained, respectively (Figure 2A). In order to perform the deconvolution, 10 of these 28 amino acids were selected, on the basis of their degree of activity in the biological assay (Figure 2A). Finally, of the possible combinations, 16 sequences were selected to perform the synthesis of the defined hexapeptides (Figure 2B). The selection of these 16 peptides was based on redundancy criteria between the physical and chemical properties of active amino acids in similar positions.

The 16 defined hexapeptides were assayed at the highest possible concentration as a function of the percentage of DMSO wherein they were dissolved. p10 showed a significant increase in the number of females born as compared to the control with DMSO at a concentration of 80 µM. Table 1 indicates the activity of the peptides of the invention. * indicates p-value < 0.05. - indicates that no females were born, in neither the control tubes and the treated tubes.

**Table 1**

| **Activity of the defined peptides obtained following deconvolution** | | |
|---|---|---|
| Nomenclature/ Sequence | Concentration | **Treated born /Control born** |
| p1 / SEQ ID NO: 1 | 80 µM | 0.3 |
| p2 / SEQ ID NO: 2 | 80 µM | - |
| p3 / SEQ ID NO: 3 | 80 µM | 0.8 |
| p4 / SEQ ID NO: 4 | 62 µM | - |
| p5 / SEQ ID NO: 5 | 25 µM | 2.0 |
| p6 / SEQ ID NO: 6 | 25 µM | - |
| p7 / SEQ ID NO: 7 | 80 µM | 1.4 |
| p8 / SEQ ID NO: 8 | 57 µM | 0.9 |
| p9 / SEQ ID NO: 9 | 80 µM | 2.0 |
| p10 / SEQ ID NO: 10 | 80 µM | 4.0* |
| p11 / SEQ ID NO: 11 | 80 µM | 0.8 |
| p12 / SEQ ID NO: 12 | 80 µM | 3.0 |
| p13 / SEQ ID NO: 13 | 80 µM | 0.8 |
| p14 / SEQ ID NO: 14 | 40 µM | 1.8 |
| p15 / SEQ ID NO: 15 | 40 µM | 0.5 |
| p16 / SEQ ID NO: 16 | 38.5 µM | 0.4 |

In order to confirm the effect of p10, the latter was subjected to dose-response assays at the following concentrations: 20 µM, 40 µM, 80 µM, 125 µM, 250 µM (Figure 3). p10 did not show any activity at 20 µM and 40 µM, whereas the activity increased at 80 µM (leading to a number of females 1.43 times greater than the control) and 125 µM (with a number of females 1.47 times greater than the control). Analysis of the data by means of a non-linear test revealed that effective dose 50 (ED50) of p10 is 70.4 µM for this phenotype. At 250 µM, the activity of the peptide decreased, possibly due to a toxic effect of the peptide at higher concentrations.

### Example 2. Study of the toxicity of p10

In order to analyse the toxicity of p10, its effect on flies with the wild genotype (OrR) was studied in a homemade nutritional medium. Since the maximum value of DMSO that the flies may tolerate in this food is not known, a first experiment was performed, which consisted of subjecting the individuals to increasing concentrations of the solvent, either from the embryo phases or from the L1 larva phase. The tolerance to DMSO in homemade food turned out to be of the order of 3-4 times greater than that determined for the instant nutritional medium from the supplier Sigma (0.3-0.4% vs 0.1%, Figure 4). The survival results obtained for the individuals treated with DMSO from the embryo phase showed a very high variability (Figure 4A), probably due to the presence of unfertilised eggs amongst the embryos selected. The individuals treated from the larva phase showed a much more homogeneous behaviour (Figure 4B). Therefore, it was decided to feed L1 larvae with increasing concentrations of p10 and the number of individuals that reached the pupa and adult phases was counted. p10 was not toxic in any of the stages at any of the concentrations assayed (67.5 µM, 125 µM, 250 µM, 500 µM and 1 mM; n: 150 in each case; Figure 4C); consequently, there could be a genotype-dependent toxic effect in the case shown in Figure 3.

### Example 3. Study of the relevance of each amino acid in the peptide sequence

In order to determine the contribution of each of the p10 residues to their activity on CTG(480), an alanine scanning experiment was performed. These experiments are based on substituting every amino acid of the peptide with alanine and keeping the rest. Alanine is a small amino acid and, in general, not very active; this is why it is used for this type of studies. Since each alanine substitution examines the contribution of an individual amino acid, this experiment makes it possible, on the one hand, to evaluate whether p10 is susceptible to optimisation (in the event that a substitution increases the efficacy of the molecule), or to determine which amino acids are important for its function (in the event that a substitution decreases the activity thereof). The sequence of p10 allows for 5 substitutions; for this reason, 5 new hexapeptides were synthesised which were assayed in relation to the lethality phenotype in *103Y-Gal4; UAS-CTG(480)*/*+* flies and commercial food. They all showed a lower activity than the original molecule, which indicates that all the amino acids in the sequence of p10 are necessary for the final activity of the peptide in this functional assay (Figure 5).

### Example 4.Validation of the activity of p10 in other tissues. p10 improves the histological defects caused by CTG(480) in the indirect flight muscles

Since the main symptoms characteristic of DM1 affect the muscle, it was decided to study the effect of p10 on this tissue in the model flies. The expression of CTG(480) under the control of the Myosin heavy-chain promoter *(Mhc-Gal4* line) causes defects at the histological level in the indirect flight muscles (IFMs), which include the loss of muscle fibres and progressive degeneration. These defects affect their function, preventing the flies from flying. Peptide p10 was assayed on the lack-of-flight phenotype using the *falling into the cylinder* method developed by Benzer (Benzer, 1973) in a homemade nutritional medium. No changes were observed in the flies' flight capacity (concentrations assayed: 62.5 µM and 125 µM; n: 117 and 77, respectively) as compared to the control with DMSO (n: 55). However, the microscope analysis of cross-sections of the thorax of model flies 3-4 days of age revealed an improvement in the muscles at the histological level (Figure 6). Since the flies' flight capacity is very sensitive to structural and metabolic changes in the IFM sarcomeres, the latter could be the cause why the histological improvement observed did not lead to a functional improvement.

In order to confirm this observation, as well as quantify the effect observed, p10 was subjected to a dose-response assay at the following concentrations: 62.5 µM, 125 µM, 250 µM and 500 µM, and semi-thin histological cuts were made in flies 10 days of age (Figure 7A-E). The quantification of the muscle area in the treated flies revealed a significant dose-dependent improvement (Figure 7F). These results also demonstrated that the effect of p10 is independent from tissue-specific factors, since p10 is active in both the brain and the muscle. At 500 µM, however, the muscles showed a reduced size and the viability of the flies decreased.

### Example 5. The endogenous expression of an L-amino acid peptide that comprises p10 suppresses phenotypes caused by CTG repeats

The administration of p10 suppressed at least part of the phenotypes caused by CTG(480). Since in the experiments initially performed in the present invention p10 is added to the food, it was not possible to determine how many molecules finally reached the cells. In order to be able to precisely control the administration of p10 and ensure the presence of the peptide in the same tissues and at the same time as the CUG(480) transcripts, transgenic flies were generated capable of expressing p10 in an endogenous and controlled manner using the Gal4/UAS system.

Thus, three different transgenes were designed, with SEQ ID NO: 17-19 (p17-p19), which encoded different peptides, all based on sequence SEQ ID NO: 10 corresponding to p10 (Figure 9), taking into consideration the codon usage bias in *Drosophila* (i.e. favouring the use of G, and in particular C, in the synonymous sites; Powell & Moriyama, 1997) and adding an initial Methionine, as well as three spacer Glycines, to sequence SEQ ID NO: 10. Moreover, upstream from the ATC initiation codon, 21 nucleotides of the 5'UTR end of the *act5C* gene of *Drosophila* which included the Kozak sequence were added, in order to enhance the expression of the transgenes.

p17 contained the direct sequence of the peptide of SEQ ID NO: 10. As discussed above, the peptides in the hexapeptide chemical library are composed of D-amino acids. The D-forms (dextrogyre) of amino acids are stereoisomers of the L-forms (levogyres) and only L-amino acids are synthesised in the cells and incorporated into proteins. Consequently, if the arrangement of the lateral chains of p10 is important for the function thereof, the latter would be lost when p17 is synthesised by *Drosophila* from L-aa. For this reason, it was decided to generate construct p18, wherein the peptide sequence of SEQ ID NO: 10 was inverted, leading to a retro-inverse peptide (Chorev & Goodman, 1995; P. M. Fischer, 2003). At the time when these transgenes were generated, the smallest constructs described in the literature encoded peptides with more than 20 amino acids, which is more than twice the size of peptides p17 and p18 (which have only ten amino acids). For this reason, construct p19 was generated, which combined the constructs of peptides p17 and p18, leading to a peptide with 19 amino acids. The three constructs were microinjected in the precursors of the germinal line of *Drosophila* embryos, leading to 10 lines of transgenic flies for each transgene.

The transgenic flies obtained were crossed with the *GMR-Gal4 UAS-CTG(480)lCyO* and *Mhc-Gal4 UAS-CTG(480)lTM6b* recombinant fly lines generated during this invention, which directed the expression of the CTG repeats in the eye and the muscle, respectively. As in the case of the *sev-Gal4 UAS-CTG(480)l+* flies, the adult *GMR-Gal4 UAS-CTG(480)*/*UAS-GFP* flies presented a strong rough eye phenotype. The co-expression of CTG(480) and p18 or p19 (but not p17) at 19°C and 21°C led to flies with eyes that were visibly less rough than those of the control individuals (Figure 11; Figure 10B-C and E-F). These results confirm that L-amino acid peptides that contain the sequence of p10 in a retro-inverse arrangement are capable of suppressing phenotypes triggered by the repeats and indicate that peptides p18 and p19 were translated and active despite their short size, since the expression thereof modified the toxicity of CTG(480). However, the opposite phenomenon occurred at 25°C and at 29°C. At 25°C, the endogenous expression of p18 or p19 enhanced the toxicity of the CTG repeats, leading to a smaller-sized eye, with loss of pigmentation and a dramatic fusion of ommatidia (Figure 10H-I). At 29°C, the crossing between *GMR-Gal4 UAS-CTG(480)lCyO* and *UAS-p1B* or *UAS-p19* flies did not produce offspring without the *CyO* balancer, which indicates that p10 reduced the viability of the *GMR-Gal4 UAS-CTG(480)* individuals at said temperature. It is worth noting that the expression of p18 and p19 by itself at 25°C and 29°C did not cause any phenotype (Figure 10G). Given that changes in temperature affect the expression of the CTG(480) transgenes and peptides p17-p19 in a different manner, since the latter are inserted in different regions of the genome, there may be a relation between the quantity of peptide and of repeats that must be reached, whereafter p10 becomes toxic.

Significantly, all the lines carrying the p18 transgene (*UAS-p18*) assayed (i.e. 5) and one of the three lines carrying the p19 transgene (*UAS-p19*) modified the phenotype of the *GMR-Gal4 UAS-CTG(480)* flies, whereas none of the five lines of construct p17 (*UAS-p17*) assayed did so. This indicates that the orientation of the lateral chains of the amino acids of p10 plays an essential role in the activity thereof.

The co-expression of CTG(480) and p18 or p19 with the *Mhc-Gal4* line at 25°C also suppressed the histological defects of the IFMs in recently ecloded adults, increasing the size of the muscular packages (Figure 12). This confirms the effect of p10 on CTG(480). Once again, only those lines that carried the p18 and p19 transgenes modified the phenotype.

### Example 6. Study of the mechanism of action of p10

The results obtained *in vivo* demonstrated an effect of p10 on the toxicity of the CTG repeats. Moreover, Figure 13 shows several hypotheses that summarise how p10 could exert its effect on the cells. In the first place, p10 could affect the levels of CUG(480) transcripts, either by interfering with the Gal4/UAS system or reducing the stability of the toxic RNAs (Figure 13A). On the other hand, p10 could bind to the CUG repeats, releasing nuclear factors sequestered thereby, such as Mbl. In this way, said proteins could once again perform their normal functions (Figure 13B). p10 could also bind to the CUG repeats, preventing these from folding to form toxic double-stranded hairpin loops (Figure 13C). Finally, p10 could be acting on other proteins or transcripts downstream from the alterations caused by the repeats, inhibiting, for example, antagonists of Mbl and, consequently, compensating for the lack of function of these proteins (Figure 13D).

In order to study each of the hypotheses proposed, it was decided to perform a number of experiments at the molecular level and *in vitro,* which are explained in detail below.

### Example 7. p10 does not affect the levels of expression of CTG(480)

In order to study whether p10 affected the expression of CTG(480), two different strategies were used. In the first place, *Mhc-Gal4*/+;*USA*-*luciferase*/+ flies were fed with the peptide at 250 µM in a homemade nutritional medium. p10 caused a slight decrease in light emission levels. However, this difference was not significant with respect to the control with DMSO (α = 0.05, T-test; Figure 14), which demonstrates that the peptide did not affect the Gal4/UAS system in a non-specific manner. However, the presence of DMSO by itself in the food of the control flies caused an increase in the levels of the luciferase protein. These results indicate that the solvent might affect the expression of this transgene, possibly due to an effect described as suppressing position-dependent variegation.

In the second place, it was determined whether p10 specifically affected the levels of CUG(480) transcripts by means of RT-PCR, starting from *Mhc-Gal4*/*+;UAS-CTG*/*+* flies fed with p10 at different concentrations. After quantifying the intensity of the bands obtained in the PCR reaction, no significant differences were detected in the levels of CUG(480) at any concentration (Figure 15), which demonstrates that p10 did not affect the quantity of transcripts.

### Example 8. Study of the similarity between the sequences of p10 and Mbl

By means of a biocomputer analysis, using the MEGA sequence comparison programme (www.megasoftware.net), it was found that the amino acids of p10 partially align with the reverse of a conserved sequence of the first zinc finger of Muscleblind proteins, a domain wherethrough the proteins bind to RNA (Figure 16). In this alignment, the Tryptophan of p10 coincides with the position of a Phenylalanine of Mbl (an amino acid that is also aromatic, apolar and hydrophobic), which mediates binding of the protein to the CUG repeats. Given this similarity, it was decided to study the effect of the peptide on the binding of Mbl to the toxic RNAs.

### Example 9. Expression and purification of the Zinc fingers of Mbl

In order to verify whether the peptide was capable of competing with Mbl in binding to the repeats, it was decided to perform *in vitro* binding assays between RNA and protein. In order to perform such experiments, it was necessary to express and purify the Mbl protein. Only the portion of Mbl corresponding to the zinc fingers thereof (MbIZF, amino acids 1-98) was used, for various reasons. In the first place, there are 7 different Mbl isoforms in *Drosophila.* Except for the MBID isoform, they all share the region that contains the two zinc fingers (Nt end) and differ in the Ct sequences. On the other hand, both the zinc fingers of MBNL1 and Mbl are sufficient to bind to the target sequences thereof.

During the cloning of the pET-15b commercial vector in a derived vector, the MblZF protein was fused to 6 Histidines in order to facilitate the purification thereof, as well as to the TEV (*Tobacco Etch Virus*) protease cleavage site. In order to find the optimal conditions for the expression of MbIZF in *E. coli,* combinations of various factors were assayed. In the first place, we started from three different bacterial strains. Strain BL21 (DE3) is a strain designed for expression systems based on the T7 bacteriophage promoter. This strain is adequate for the expression of non-toxic recombinant proteins. In the event that the protein to be expressed is toxic, strain BL21 pLys (DE3) of *E.coli* has the advantage of constitutively expressing low levels of the T7 lysozyme, which reduces the baseline expression of recombinant genes by inhibiting the levels of T7 RNA polymerase. Finally, strain BL21 pLys (DE3) Codon + contains genes that encode tRNAs for human codons and which bacteria have at very low levels. In the second place, zinc fingers domains are nucleic acid binding domains that bind one atom of the zinc ion between their Cysteine and Histidine residues. The zinc metal is crucial for the stability of this type of domains, since, in the absence thereof, the zinc fingers are deployed and lose the capacity to bind to their targets. For this reason, two different concentrations of ZnCl₂ were assayed in all the culture media used throughout the expression of the MbIZF protein (50 µM and 100 µM). Moreover, proteins with zinc fingers tend to be quite insoluble and unstable molecules. For this reason, we assayed two different induction temperatures, 30°C and 16°C. Amongst all the combinations tested, we managed to obtain a high proportion of soluble MbIZF when using strain BL21 (DE3), a 50 µM concentration of ZnCl₂ and an induction temperature of 16°C with 1 mM of IPTG (Figure 17A).

The purification process was performed by affinity chromatography in an FPLC system, throughout which the presence of zinc in the medium was maintained. The elution peak for the protein took place at approximately 300 mM of imidazole (Figure 17B). The total protein concentration obtained was 2.73 mg/ml (198 µM; Figure 17C), which means a yield of 1.4 mg of protein/I of culture. In subsequent purification cycles, this value did not change in a significant manner.

In order to verify that the purified protein was in its native conformation following the purification, its circular dichroism (CD) spectrum was analysed. Circular dichroism is an electronic absorption spectroscopy technique based on the differential absorption by the molecule of right- and left-circularly polarised light beams. Since different proteins have a different circular dichroism spectrum, this technique makes it possible to identify the conformation of different molecules by comparison with a theoretical spectrum (Figure 18B). The circular dichroism spectrum obtained for MBIZF in phosphate buffer is similar to that described for the zinc fingers of MBNL1, with a pronounced peak at 203 nm, which denoted a lack of structure, and a weak peak at 220 nm, which indicates an alpha helix (Figure 18A). In the case of MbIZF, this pattern was conserved, although the values were slightly shifted to the right (Figure 18C). Finally, in order to determine whether MbIZF was found as a monomer in solution or forming complexes, we performed molecular exclusion chromatography (Figure 18D). Molecular exclusion chromatography is a column chromatography method whereby the molecules separate as a function of their molecular weight. The exclusion volume obtained for MblZF corresponded to a molecular weight of 13.4 KDa, equivalent to the size of the protein in monomer form. The chromatogram revealed a single elution peak, which demonstrates that MblZF does not form complexes with itself in solution, at least under the assay conditions.

### Example 10. p10 binds to CUG repeats in vitro but does not compete with MbIZF

Amongst the different techniques for the *in vitro* study of interactions between proteins and nucleic acids, fluorescence polarisation (FP) assays are worth highlighting for their rapidity and sensitivity. This technique is based on the changes in rotational movement of fluorescent molecules in suspension. Thus, when a polarised light beam excites a fluorophore conjugated to a small molecule, the latter undergoes rotational diffusion faster than the time needed for the emission of light, which results in a random arrangement of the molecule at the time of fluorescence emission (depolarisation). However, rotation of the molecule becomes slower when the viscosity of the medium or the molecular volume change, increasing the polarisation of the light emitted. Thus, by measuring the polarisation changes in a molecule, it is possible to detect the binding between the latter and another molecule added to the medium (Figure 19). In the present invention, these molecules were p10, MblZF and 23 CUG repeats conjugated to the carboxyfluorescein fluorophore (FAM-(CUG)23).

In order to prepare the FP experiment, in the first place two different concentrations of the FAM-(CUG)23 RNA (6 nM and 60 nM) were assayed. In both cases, the RNA was detected and a significant increase was observed in the fluorescence polarisation values upon adding MblZF (p-value < 0.0001), which indicates that binding had taken place. In order to reduce the use of both the probe and the protein, it was decided to work at a FAM-(CUG)23 concentration of 6 nM. The interaction observed between MbIZF and FAM-(CUG)23 was proportional to the quantity of protein (Figure 20B). Statistical analysis of the binding curve led to a theoretical IC50 of 900 nM. In order to check the specificity of this binding, competition studies were performed with an unlabelled RNA, (CUG)23 (1:10, 1:100 and 1:200 with respect to FAM-(CUG)23). In all cases, competition was observed at all the unlabelled probe concentrations assayed (Figure 20A). p10 also caused a slight increase in the polarisation of FAM-(CUG)23 (p-value: 0.0176; Figure 20D). However, the addition of increasing quantities of p10 did not lead to a binding curve, possibly due to the small size of p10 (Figure 20D). The co-addition of p10 and MblZF to FAM-(CUG)23 caused a greater increase in the polarisation of RNA than that caused by the addition of MbIZF alone (p-value: 0.0158; Figure 20C). This result indicates that both molecules may bind to RNA without interfering with the binding of the other. However, the increase in the polarisation values following the co-addition of MbIZF and p10 was lower than the sum of the increases caused by both separately (ΔmP(Mbl): 99.5; ΔmP(p88): 21.75; ΔmP(Mbl+p88): 108.25), which does not rule out the existence of partial competition.

The changes on the RNA caused by p10 in the FP assay are small, probably due to the low molecular weight of the molecule (less than 900 Da). For this reason, it was decided to supplement the study with gel retardation experiments. These assays are based on the differences in electrophoretic mobility between stable protein-nucleic acid complexes and their components separately. Since the FAM-(CUG)23 RNA was available, it was decided to prepare a fluorescence gel retardation assay. After determining the optimal quantity of RNA for detection (60 nM; Figure 21A), the binding between the latter and MbIZF was studied. Said binding caused both a decrease in the intensity of the band corresponding to the free RNA in the gel and the appearance of a signal in the interior of the gel wells (bound RNA). However, no retardation bands were observed in the gel, which indicates that all the complexes formed following the binding were retained in the well. The signal in the interior of the well decreased when the binding competed with the unlabelled (CUG)23 probe (1:100), which demonstrates the specificity of the interaction. Moreover, binding did not take place when the fluorescent RNA was incubated with the heat-denatured MblZF protein, nor when the reaction took place between FAM-(CUG)23 and a different protein (collagen α3) at the same concentration as MbIZF, once again confirming the specificity of the binding (Figure 21 B).

In order to rule out the possibility that FAM-(CUG)23 could bind to a 6-Histidine tag of MbIZF, they were eliminated by means of digestion with TEV protease (Figure 22A-C). The MblZF protein without Histidines (MblZF^{ΔHis}) maintained the RNA-binding capacity (Figure 22D).

Retention of the complexes formed between MblZF and FAM-(CUG)23 in the well could be caused by the formation of large-size aggregates between the RNA and the protein. In order to reduce the size of said complexes, we used an RNA with 4 CUG repeats (FAM-(CUG)4). The zinc fingers of MBNL1 may bind to CUG repeats formed by a minimum of 4 triplets, provided that formation of the loop is facilitated by adding 4 non-complementary nucleotides towards the middle of the sequence. As shown in Figure 21C, MblZF bound to FAM-(CUG)4, but the complex formed was equally retained in the well.

The isoelectric point (Ip) of MblZF is 9.5. The pH of the electrophoresis buffer used in our assays is 8.5, which means that the total charge of MblZF in the gel is positive. Retardation gels are non-denaturing gels; consequently, there is no SDS present in the medium to confer a negative charge to the molecule such that it migrates towards the positive pole. In general, this is usually not a disadvantage in gel retardation assays, since the RNA has a negative charge and is capable of dragging the complex during the electrophoresis. However, it was decided to verify that FAM-(CUG)23 could mobilise MbIZF, by running the complex in a horizontal 2.5% agarose gel with the wells placed in the middle and staining it with Coomassie (Figure 23A). In this experiment, the MblZF protein alone migrated towards the negative pole, in the direction opposite to the protein molecular weight marker. When MblZF was incubated with FAM-CUG(23), the quantity of protein that migrated towards the negative pole (probably unbound protein) decreased, but still no signal appeared towards the positive side of the gel, even when the electrophoresis was performed at a pH of 10.5, greater than the lp of MblZF (Figure 23B).

In order to confer the necessary negative charge, the FAM-(CUG)23-MblZF complex was fixed by cross-linking with formaldehyde (FA) and the reaction was run in a polyacrylamide gel under denaturing conditions (in the presence of SDS; Figure 23C) or, alternatively, by adding Serva Blue G, which confers a charge to the proteins without denaturing them. In no case did the complex penetrate into the interior of the gel.

In order to determine whether p10 could bind to CUG RNA molecules, it was decided to perform a similar gel retardation experiment between the peptide and FAM-(CUG)23. p10 bound to the RNA, with part of the complex being retained in the well, as described for MbIZF. However, in this case a retardation band was observed in the interior of the gel (Figure 24A). p10 bound to FAM-(CUG)23 at high peptide concentrations (500 µM and higher, Figure 24B), which indicates a low affinity between both molecules, at least under the assay conditions. This affinity decreased upon decreasing the size of the repeats (FAM-(CUG)4, Figure 24C). In order to determine whether p10 could compete with MblZF for binding to RNA, both were incubated in the presence of FAM-(CUG)23. p10 did not prevent the binding of MbIZF, since in this case all the RNA was retained in the well (Figure 24D). This could be due either to a synergic effect between the protein and the peptide or to an additive effect if both should bind to different RNA sites.

### Example 11. p10 binds to CUG repeats with a higher affinity than to other sequences

In order to study the specificity of the binding observed between p10 and the CUG repeats, two experiments were performed. In the first place, the five hexapeptides generated during the alanine scanning were assayed. None of them significantly bound to FAM-(CUG)23 (Figure 25A), which demonstrates that all the p10 residues are necessary for interaction with the RNA. Interestingly, these peptides did not suppress the toxicity of the CTG(480) transgene in *Drosophila,* which indicates that the capacity of p10 to bind to CUG repeats is necessary for its *in vivo* activity. In the second place, we studied the effect of p10 on other RNA and DNA sequences, both double-stranded and single-stranded, fluorescently labelled with carboxyfluorescein (FAM) (Figure 25C). Said molecules included: (1) a single-stranded RNA made up of 19 nt from the 3'UTR region of the *DMPK* gene (ssRNA), (2) an RNA made up of 4 CUG repeats fused to the single-stranded sequence of *DMPK* (ds+ssRNA), (3) an RNA capable of forming a perfect double-stranded hairpin loop composed of 4 CAG·CUG repeats (dsRNA), (4) a DNA made up of 4 CTG repeats (dsDNA) and (5) a DNA corresponding to the same 19 nt of the 3'UTR region of the *DMPK* gene. p10 bound in all cases (Figure 25B). Moreover, an unlabelled mutated RNA with sequence (GUC)4 (whereto the Zinc fingers of human MBNL1 proteins do not bind) and tRNA shifted the binding between p10 and FAM-(CTG)23. Overall, these data indicate that the binding of p10 to RNA was sequence-specific (since other hexapeptides showed a different behaviour) and that the peptide could bind to more than a single type of nucleic acid.

However, it was possible for p10 to bind to each of the sequences studied with a different affinity. Since the peptide-RNA and peptide-DNA complexes detected in the gel retardation experiments were retained in the well, it was not possible to determine the dissociation constants (K_{d}) for each interaction. In order to quantify the binding between p10 and said nucleic acids, as well as rule out the possibility that the peptide were binding to carboxyfluorescein in all cases, it was decided to prepare an intrinsic fluorescence extinction experiment for the Tryptophan present in the sequence of p10. Tryptophan has a maximum absorption at a wavelength of 280 nm and a maximum emission peak between 300 and 350 nm (depending on the solvent polarity). The fluorescence of this residue changes when the conformation of the protein that comprises it is modified by, for example, binding to another molecule. Thus, increasing quantities of the DMPK-CUG4, DMPK and CAG-CUG4 RNA molecules, as well as CTG4 and DMPK DNAs, were incubated with a fixed quantity of p10 (5 µM), and the changes in the fluorescence emission of the peptide were measured at 351 nm. In all cases, the signal decreased proportional to the quantity of RNA or DNA added to the medium, which confirms the previously observed binding. However, representation in a straight line of the fluorescence emitted for each concentration point relative to the fluorescence of the free peptide led to straight lines with different slopes, which indicates that the fluorescence extinction rate (and, consequently, the binding affinity) was different for the different nucleic acids (Figure 26). The slope of the curve was significantly greater for the DMPK-CUG4 molecule (-0.034 ± 0.003) than for CTG4 (-0.019 ± 0.005) and DMPK (RNA: -0.024 ± 0.003 and DNA: -0.028 ± 0.002) (p-value < 0.01). However, for CAG-CUG RNA (-0.028 ± 0.003), this difference was not statistically significant (p-value > 0.1; Figure 26B). The DMPK-CUG4 molecule contained the CUG repeats in their DMPK environment. Overall, this indicated that there was target binding selectivity on the part of the peptide; therefore, it was possible for it to preferably bind to the toxic RNAs *in vivo.*

### Example 12. p10 causes a conformational change in the CUG repeats

In order to study possible changes in the secondary structure of RNA induced by p10, in the first place we performed a circular dichroism (CD) experiment, using an RNA with unlabelled CUG repeats (CUG60). The circular dichroism of nucleic acids is caused by the stacking of the bases that make up the sequence thereof. Consequently, when a molecule binds to RNA, affecting the structure thereof, a change is detected in its CD spectrum. Incubating the CUG60 RNA (1 µM) with the MblZF protein (1 µM and 1.5 µM) reduced the intensity of the signal emitted by the latter, although it did not shift the peak wavelength in the spectrum (Figure 27A), which indicates that the binding of MbIZF affected the packaging of the RNA bases. When we incubated the RNA with increasing concentrations of the peptide of SEQ ID NO: 10 (0.1 µM, 0.5 µM, 1 µM, 10 µM and 20 µM), a similar effect was observed (Figure 27B). During the readings, the RNA was kept in the cuvette inside the dichrograph at 10°C. Throughout the 3 hours of each experiment, it was possible for the RNA to have been degraded; this would explain the decrease in the intensity of the signal observed. In order to rule out this possibility, the CD spectrum of CUG60 RNA was measured at times 0 and 3 h, having kept the CUG60 RNA at 10°C at all times. At 3 hours, the spectrum of CUG60 did not show any changes with respect to the spectrum obtained at time 0, which demonstrates that the effects observed were specific (Figure 27D). Moreover, alanine scanning peptides p29.1 (0.5 µM and 1 µM) and p29.4 (1 µM) did not change the RNA spectrum (Figure 27E and 27F), thereby confirming the binding specificity. Finally, when MbIZF (0.5 µM) was added to the RNA, followed by p10 (0.5 µM), or vice-versa, no increase in the CD signal was observed, which suggests that the changes produced in the RNA by one of them are not reversible by the subsequent addition of the other.

These results demonstrate that p10 reduces the stacking of the RNA bases and, therefore, affected the secondary conformation of the molecule. In order to confirm whether said changes cause a loss of the hairpin loop structure, an experiment was performed using an RNA composed of 23 CUG repeats, wherein a guanine had been substituted with its analogue 2-aminopurine (2-AP-(CUG)23). When 2-AP is in a single-stranded environment, it emits fluorescence at 375 nm. However, said emission is significantly extinguished when the molecule is forming a part of a double helix. At low concentrations of MblZF and p10, for which binding had been detected in the CD and Trp-FQ experiments (RNA:protein ≤ 1:5), neither of them caused changes in the fluorescence of 2-AP (Figure 28A-B). However, upon increasing the concentration of p10 to 100 µM (RNA:peptide 1:100), the 2AP-(CUG)23 RNA underwent a conformational change, from double-stranded to single-stranded, with a 2.9-fold increase in the fluorescence emission of the molecule (Figure 28B). In order to confirm the specificity of this effect, the same experiment was performed, incubating the alanine scanning peptides (payawe and ppyawa) with the 2-AP-(CUG)23 RNA at the same concentration as p10 (100 µM). None of these peptides caused changes in the fluorescence emission; neither did DMSO by itself (Figure 28C). Therefore, these results indicate that p10 is capable of destabilising the double strand formed by the CUG repeats and confirm that MblZF and the peptide bind to RNA in a different manner.

### Example 13. Validation of the effect of p10 in a DM1 mouse model. Study of the toxicity of p10 in mammals

In order to study the possible toxic effect of p10 in mammals and establishing the maximum tolerable dose for subsequent assays, the peptide was administered to mice from the *FVB* wild strain. Due to the low absorption of peptides by the intestinal walls, it was decided to perform intramuscular injections in the anterior tibial muscle of animals 4-5 weeks of age, using four different doses (0.5 µg, 1 µg, 10 µg and 100 µg), with a total of 6 mice per dose (3 females and 3 males). 4 weeks after the injection, the animals were sacrificed and a visual autopsy was performed, jointly with a histomorphological study of the muscle and a blood test (Figure 31). As a control, animals injected with the same quantity of DMSO (0.2% for the 0.5-pg and 1-µg doses, and 2% for the 10-µg and 100-µg doses) were used.

The visual autopsy and the histological analysis of the muscles of the treated animals did not reveal significant differences with respect to the controls with DMSO. In some cases, signs of slight mixedematosis (accumulation of liquid) were observed and, occasionally, a mild inflammation, probably caused by the injection itself (Figure 31). However, the blood tests did show differences between the treated animals and the control. As a marker of renal activity, we measured the quantity of bile acids, urea and creatinine in the blood. The creatine phosphokinase (CPK) enzyme was used as a marker of damage to the skeletal muscle and the heart, and alkaline phosphatase (AP), gamma-glutamyl transpeptidase (GGT) and glutamate pyruvate transaminase (GPT) were used as markers of hepatic damage. All the animals (including the control) showed high values of urea and AP with respect to standard values. Moreover, the control animals subjected to 2% DMSO showed abnormally high values of GPT and CPK. This could be due to the toxicity of DMSO or to the injection itself. However, the 10-µg and 100-µg doses showed even higher values of urea and GPT in the blood than the controls. Urea is the final result of protein metabolism. It is formed in the liver and eliminated in the urine. If the kidney is not functioning correctly, urea accumulates in the blood. GPT is an enzyme that is present in the liver at a high concentration and, to a lower extent, in the kidneys, the heart and the muscles. When there is an injury in these organs, the enzyme is released into the blood and appears high in tests. Moreover, it is worth noting that two of the mice treated with the 100-µg dose died approximately 11 days after the injection (p/i), which confirms the toxicity of p10 when it exceeds a threshold value. It was possible to extract blood from one of these two animals just before it died. This mouse presented extremely high values of urea, creatinine, CPK and GGT in the blood (e.g. a value for CPK 28 times greater than the upper limit of the standard values and 15 times greater than the control with DMSO).

### Example 14. p10 reverts the splicing defects of the Serca1 and Tnnt3 transcripts in HSA ^{LR} mice

p10 suppressed the effect of CTG(480) in the CNS, the muscle and the eye of *Drosophila* and bound to CUG repeats *in vitro.* In order to validate the relevance of these results in DM1 mammal models, it was decided to assay p10 in *HSA^{LR}* mice. These mice express 250 CUG repeats in a heterologous transcript, human skeletal actin (HSA), and reproduce most of the symptoms of DM1, including defects in the alternative splicing of transcripts. One of the clearest examples of altered splicing in both patients and *HSA^{LR}* mice is that of exon 22 of the Ca⁺²-dependent ATPase *Serca1* transcripts. In the healthy population, said exon is excluded in foetal forms and included in adults. However, in both patients and *HSA^{LR}* mice, exon 22 is excluded throughout life and, therefore, a foetal pattern is maintained in adult individuals. The Tnnt3 protein is found in the sarcomeres of fast-twitch muscle fibres in the skeletal muscle. Its transcripts undergo alternative splicing of the so-called foetal exon F. In the healthy population, this exon is absent in adult individuals, whereas in DM1 patients and *HSA^{LR}* mice, the foetal exon is maintained.

In order to determine whether p10 could revert the splicing defects of *Serca1* and *Tnnt3* in *HSA^{LR}* mice, intramuscular injections of two doses of the peptide were administered: 0.5 µg and 10 µg. The treated animals were sacrificed 1 week, 2 weeks and 4 weeks after the injection, and the anterior tibial muscle of both the right leg (injected with p10) and the left leg (injected with 0.2% DMSO in saline serum for the 0.5-µg dose and with 2% DMSO in saline serum for the 10-µg dose) were dissected. As a control, we used *FVB* animals and *HSA^{SR}* animals (which express 5 CUG repeats in human skeletal actin transcripts) injected in both legs in a similar manner, as well as *HSA^{LR}* mice which had been injected with serum with DMSO in both extremities.

The RT-PCR analysis of the *Serca1* and *Tnnt3* transcripts of animals treated with 0.5 µg did not reveal significant changes in the inclusion of exons 22 and foetal F, respectively, at any of the times assayed (gels not shown). However, in 2 of 5 of the animals injected with 10 µg, p10 reverted the splicing defects in both cases (Figure 32A-C). Given the complex band pattern obtained for the *Tnnt3* transcripts, we could not precisely quantify the increase in the exclusion percentage of foetal exon F induced by p10. In the case of *Serca1,* p10 increased the inclusion percentage of exon 22 by 1.3% (1 week p/i, p-value > 0.05), 25.9% (2 weeks p/i, p-value < 0.05) and 38.3% (4 weeks p/i, p-value < 0.01) (Figure R40D). Finally, p10 did not affect the splicing of the muscular transcripts of the *Capzb* gene, which were used as a specificity control, since the processing thereof is not altered in the model *HSA^{LR}* mice (Figure 32A-C).

Analysis of the processing of the *Serca1* transcripts in the right leg (injected with p10) and the left leg (injected with serum and 2% DMSO) of the same animal at 4 weeks p/i revealed that, in the untreated extremity, the inclusion percentage of exon 22 was greater than that shown by the *HSA^{LR}* animals injected with DMSO in both legs (Figure 33). This indicates that the quantity of p10 that reached the anterior tibial muscle of the left extremity through the blood was sufficient to partially revert the splicing defects in a systemic manner.

### Example 15. p10 reverts histological defects in the muscle of HSA^{LR} mice

The main histological-level characteristic of both DM1 and *HSA^{LR}* animals is the presence of central nuclei in the muscle fibres. The injection of 0.5 µg and 10 µg in the anterior tibial muscle of these animals significantly reduced the percentage of cells with central nuclei as compared to the *HSA^{LR}* animals treated with DMSO, whereas it did not produce any changes in the *FVB* mice (Figure 34). This effect was more marked 4 weeks after the injection, which indicates that it is a slow process.

Overall, the results obtained in the model mice validate the therapeutic potential of p10 in mammals, opening up new study pathways in the search for treatments for DM1 and DM2 and SCA8.

### Example 16. Comparative assay of several peptides of the invention (p10, p11, p5, p12 and p15) in the destructuration or opening of the hairpin loops formed by repeats different than CUG repeats, such as AAG (control), CGG, CCUG and CAG

As may be observed in Figure 29, in this example we use the 2-aminopurine assay in order to assay 4 peptides (p11, p5, p12 and p15) in addition to p10, obtained from the deconvolution of the combinatorial peptide library explained in this invention. The results indicate a correlation between the activity obtained with the peptides (Table 1) and the capacity of the CUG23 probe to destructurate secondary structures and become single-stranded. The concentration used in all cases is 100 µM. Therefore, this experiment demonstrates that, although p10 is the preferred peptide, since it is the most active, the rest of the peptides of Table 1, particularly p11, p5, p12 and p15, also present significant levels of specificity and efficacy.

On the other hand, as represented in Figure 30, the peptides of the invention (particularly p10) have different binding affinities to hairpin loops formed by repeats other than CUG repeats, such as, for example AAG, CGG, CCUG and CAG. In this example, we tested p10 and its capacity to bind to repeats different from CUG repeats, but which are also involved in diseases where the toxicity of RNAs with long repeats has been described. The tryptophan extinction assay was used, using, on the one hand, p10 + AAG, CGG, CCUG and CAG (AAG is used as a control because it does not form secondary structures) and, on the other hand, the same experiment using one of the inactive peptides obtained following the alanine scanning (ppyawa). The results indicate that p10 presents levels of binding to other types of repetitive sequences that are similar to those previously observed with DMPK-CUG4 (highly specific in its binding to p10) (Figure 26). This binding was greater in the presence of CGG and CCUG repeats involved in the FXTAS and DM2 diseases, respectively. Three assay points were used (3 different probe concentrations), with two measurements in each. The results were normalised to the signal offered by p10 alone. The results of p10 as compared to one of the alanine scanning peptides (ppyawa) (without activity) showed a completely different response to the presence of different RNA repeats. The decrease in intensity shown by p10 in the presence of almost all the types of repeats dramatically changes direction (increase in intensity) when peptide ppyawa is used. This increase in intensity, instead of a decrease thereof, is also indicative of the type of binding to the probes, which suggests that the efficacy of p10 is closely linked to the type of interaction with the secondary structures formed by different types of repeats. A single assay point is shown (probe at 10 µM). The results are shown non-normalised in order to be able to include the initial value of the peptides tested.

### BIBLIOGRAPHY

- Garcia-Lopez, A., Monferrer, L., Garcia-Alcover, I., Vicente-Crespo, M., Alvarez-Abril, M. C., & Artero, R. D. (2008). Genetic and chemical modifiers of a CUG toxicity model in Drosophila. PLoS One, 3, e1595.
- Mankodi, A., Logigian, E., Callahan, L., McClain, C., White, R., Henderson, D., et al. (2000). Myotonic dystrophy in transgenic mice expressing an expanded CUG repeat. Science, 289, 1769-1773.

## Claims

1. Compound that comprises a hexapeptide with Formula I, pharmaceutically acceptable salts, derivatives or stereoisomers thereof:
**A-B-C-D-E-F** (I)
where:
**A** may be any of amino acids **c** or **p**,
**B** may be any of amino acids **p** or **q**,
**C** is amino acid **y**,
**D** may be any of amino acids **a** or **t**,
**E** may be any of amino acids **q** or **w**, and
**F** is amino acid **e**.

2. Compound according to claim 1, selected from the group: SEQ ID NO: 1 to SEQ ID NO: 19.

3. Compound according to claims 1 or 2, which consists of a hexapeptide of sequence SEQ ID NO: 10.

4. Compound according to claims 1 or 2, which consists of a hexapeptide selected from: SEQ ID NO: 5, SEQ ID NO: 11, SEQ ID NO: 12 and SEQ ID NO: 15.

5. Compound according to claims 1 to 4, **characterised in that** the amino acids that form a part of Formula I are L-amino acids, D-amino acids or mixtures thereof.

6. Compound according to any of the preceding claims, which comprises a hexapeptide with Formula I in a direct or retro-inverse configuration.

7. Compound according to any of the preceding claims, for use as a medicament.

8. Compound according to any of claims 1 to 6, for use in the prevention and/or treatment of diseases the etiology whereof is based on the presence of toxic transcripts that comprise CUG, CCUG, CGG, CAG and AAG repeats included in the group formed by: DM1, SCA8, DM2, FXTAS, HD and FA.

9. Use of at least one compound according to claims 1 to 6 for the preparation of a pharmaceutical composition designed for the prevention and/or treatment of diseases the etiology whereof is based on the presence of toxic transcripts that comprise CUG, CCUG, CGG, CAG and AAG repeats, included in the group formed by: DM1, SCA8, DM2, FXTAS, HD and FA.

10. Pharmaceutical composition that comprises at least one compound according to claims 1 to 6.

11. Method for the prevention and/or treatment of diseases the etiology whereof is based on the presence of toxic transcripts that comprise CUG, CCUG, CGG, CAG and AAG repeats, included in the group formed by: DM1, SCA8, DM2, FXTAS, HD and FA, which comprises administering to a patient a therapeutically effective quantity of at least one compound according to claims 1 to 6, or the composition according to claim 10.


SEQUENCE LISTING

<110>   UNIVERSITAT DE VALENCIA
<110>   FUNDACIÓN DE LA COMUNIDAD VALENCIANA CENTRO DE INVESTIGACIÓN
PRÍNCIPE FELIPE
<110>   CONSEJO SUPERIOR DE INVESTIGACIONES CIENTÍFICAS

<120> COMPOUNDS TO BE USED IN THE TREATMENT OF DISEASES BASED ON THE
EXPRESSION OF TOXIC TRANSCRIPTS

<130>  EP-05672

<160>  19

<170>  PatentIn version 3.3

<210>  1
<211>  6
<212>  PRT
<213>  Artificial

<220>
<223>  Hexapeptide 1 (p1)

<400>  1

Cys Pro Tyr Ala Gln Glu
1               5


<210>  2
<211>  6
<212>  PRT
<213>  Artificial

<220>
<223>  Hexapeptide 2 (p2)

<400>  2

Cys Pro Tyr Ala Trp Glu
1               5


<210>  3
<211>  6
<212>  PRT
<213>  Artificial

<220>
<223>  Hexapeptide 3 (p3)

<400>  3

Cys Pro Tyr Thr Gln Glu
1               5


<210>  4
<211>  6
<212>  PRT
<213>  Artificial

<220>
<223>  Hexapeptide 4 (p4)

<400>  4

Cys Pro Tyr Thr Trp Glu
1               5


<210>  5
<211>  6
<212>  PRT
<213>  Artificial

<220>
<223>  Hexapeptide 5 (p5)

<400>  5

Cys Gln Tyr Ala Gln Glu
1               5


<210>  6
<211>  6
<212>  PRT
<213>  Artificial

<220>
<223>  Hexapeptide 6 (p6)

<400>  6

Cys Gln Tyr Ala Trp Glu
1               5


<210>  7
<211>  6
<212>  PRT
<213>  Artificial

<220>
<223>  Hexapeptide 7 (p7)

<400>  7

Cys Gln Tyr Thr Gln Glu
1               5


<210>  8
<211>  6
<212>  PRT
<213>  Artificial

<220>
<223>  Hexapeptide 8 (p8)

<400>  8

Cys Gln Tyr Thr Trp Glu
1               5


<210>  9
<211>  6

<212>  PRT
<213>  Artificial

<220>
<223>  Hexapeptide 9 (p9)

<400>  9

Pro Pro Tyr Ala Gln Glu
1               5


<210>  10
<211>  6
<212>  PRT
<213>  Artificial

<220>
<223>  Hexapeptide 10 (p10)

<400>  10

Pro Pro Tyr Ala Trp Glu
1               5


<210>  11
<211>  6
<212>  PRT
<213>  Artificial

<220>
<223>  Hexapeptide 11 (p11)

<400>  11

Pro Pro Tyr Thr Gln Glu
1               5


<210>  12
<211>  6
<212>  PRT
<213>  Artificial

<220>
<223>  Hexapeptide 12 (p12)

<400>  12

Pro Pro Tyr Thr Trp Glu
1               5


<210>  13
<211>  6
<212>  PRT
<213>  Artificial

<220>
<223>  Hexapeptide 13 (p13)

<400>  13

Pro Gln Tyr Ala Gln Glu

1                 5


<210>  14
<211>  6
<212>  PRT
<213>  Artificial

<220>
<223>  Hexapeptide 14 (p14)

<400>  14

Pro Gln Tyr Ala Trp Glu
1               5


<210>  15
<211>  6
<212>  PRT
<213>  Artificial

<220>
<223>  Hexapeptide 15 (p15)

<400>  15

Pro Gln Tyr Thr Gln Glu
1               5


<210>  16
<211>  6
<212>  PRT
<213>  Artificial

<220>
<223>  Hexapeptide 16 (p16)

<400>  16

Pro Gln Tyr Thr Trp Glu
1               5


<210>  17
<211>  10
<212>  PRT
<213>  Artificial

<220>
<223>  Peptide 17 (p17)

<400>  17

Met Gly Gly Gly Pro Pro Tyr Ala Trp Glu
1               5                   10


<210>  18
<211>  10
<212>  PRT
<213>  Artificial

<220>

<223>  Peptide 18 (p18)

<400>  18

Met Gly Gly Gly Glu Trp Ala Tyr Pro Pro
1               5                   10

<210>  19
<211>  19
<212>  PRT
<213>  Artificial

<220>
<223>  Peptide 19 (p19)

<400>  19

Met Gly Gly Gly Pro Pro Tyr Ala Trp Glu Gly Gly Gly Glu Trp Ala Tyr Pro Pro
1               5                   10                  15

1